(19) Europäisches Patentamt
European Patent Office
Office européen des brevets

(11) Numéro de publication: **0 115 979 B1**

# (12) FASCICULE DE BREVET EUROPEEN

(45) Date de publication du fascicule du brevet: **06.08.86**

(51) Int. Cl.⁴: **C 07 D 513/04**, A 61 K 31/435 // (C07D513/04, 277:00, 209:00)

(21) Numéro de dépôt: **84400057.0**

(22) Date de dépôt: **12.01.84**

(54) **Nouveaux dérivés du 1H,3H-pyrrolo (1,2-c) thiazole leur préparation et les médicaments qui les contiennent.**

(30) Priorité: **13.01.83 FR 8300453**

(43) Date de publication de la demande: **15.08.84 Bulletin 84/33**

(45) Mention de la délivrance du brevet: **06.08.86 Bulletin 86/32**

(84) Etats contractants désignés: **AT BE CH DE FR GB IT LI LU NL SE**

(56) Documents cités: **Néant**

(73) Titulaire: **RHONE-POULENC SANTE, Les Miroirs 18 Avenue d'Alsace, F-92400 Courbevoie Cedex (FR)**

(72) Inventeur: **Fabre, Jean-Louis, 9, rue Fagon, F-75013 Paris (FR)**
Inventeur: **Farge, Daniel, 30, rue des Pins Sylvestres, F-94320 Thiais (FR)**
Inventeur: **James, Claude, 31 bis, rue Gambetta, F-75020 Paris (FR)**
Inventeur: **Lavé, Daniel, 72 Bld de la Villette, F-75019 Paris (FR)**

(74) Mandataire: **Le Goff, Yves et al, RHONE-POULENC RECHERCHES Brevets Pharma 25, Quai Paul Doumer, F-92408 Courbevoie (FR)**

EP 0 115 979 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouveaux dérivés du 1H,3H-pyrrolo[1,2-c]thiazole de formule générale:

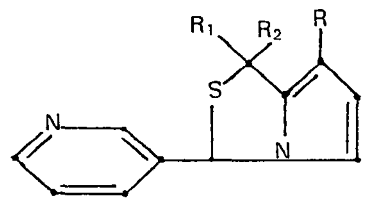

(I)

leur préparation, leurs sels et les compositions médicinales qui les contiennent.

Dans la formule générale (I), $R_1$ et $R_2$, identiques ou différents, représentent un atome d'hydrogène ou un radical alcoyle et

a) soit R représente un radical cyano ou alcoylcarbonyle

b) soit R représente un radical de formule générale:

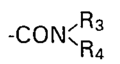

(II)

dans laquelle

— ou bien $R_3$ représente un atome d'hydrogène et $R_4$ représente un radical amino, alcoylamino, dialcoylamino, phénylamino ou diphénylamino,

— ou bien $R_3$ et $R_4$, identiques ou différents, représentent un atome d'hydrogène ou un radical alcoyle contenant 1 à 5 atomes de carbone, ou phényle éventuellement substitué,

— ou bien $R_3$ représente un atome d'hydrogène et $R_4$ représente un radical pyridyle ou alcoyle contenant 1 à 5 atomes de carbone substitué par un radical carboxy, amino, alcoylamino, dialcoylamino, morpholino, pipéridino, pyrrolidinyle-1, pipérazinyle-1 (éventuellement substitué en position 4 par un radical alcoyle, pyridyle, phényle éventuellement substitué ou benzyle éventuellement substitué), phényle éventuellement substitué, pyridyle ou imidazolyle,

— ou bien $R_3$ et $R_4$ forment ensemble un radical imidazolyle ou un hétérocycle à 5 ou 6 chaînons pouvant contenir en outre un autre hétéroatome tel que l'oxygène, le soufre ou l'azote éventuellement substitué par un radical alcoyle, alcoyloxycarbonyle, hydroxyalcoyle, aminoalcoyle, alcoylaminoalcoyle, dialcoylaminoalcoyle, pyridyle, pyrimidinyle, pyrazinyle, phényle, éventuellement substitué ou benzyle éventuellement substitué

c) soit R représente un radical de formule générale:

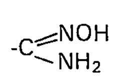 (III) ou 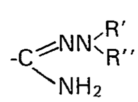 (IV)

dans laquelle R' et R'', identiques ou différents représentent un radical alcoyle,

étant entendu que dans les définitions précédentes et celles qui seront données par la suite les radicaux alcoyle et portions alcoyle sont en chaîne droite ou ramifiée et contiennent, sauf mention spéciale, 1 à 4 atomes de carbone, que les radicaux phényle et benzyle substitués sont, sauf mention spéciale, les radicaux phényle et benzyle portant un atome d'halogène ou un radical alcoyle, alcoyloxy, alcoylthio, trifluorométhyle ou dialcoylylamino, et que l'invention concerne également les formes tautomères des produits cités lorsque R représente un radical de formule générale (III) ou (IV).

Selon l'invention, les produits de formule générale (I) dans laquelle R représente un radical cyano et $R_1$ et $R_2$ sont définis comme précédemment, c'est-à-dire les produits de formule générale:

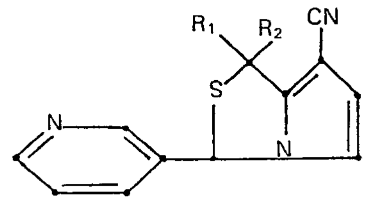

(V)

peuvent être préparés par action du chloro-2 acrylonitrile de formule:

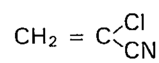

(VI)

sur un produit de formule générale:

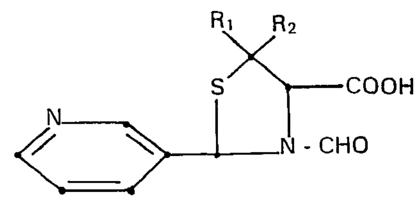

(VII)

La réaction s'effectue généralement dans l'anhydride acétique par chauffage à une température comprise entre 80° et 130°C.

Les produits de formule générale (VII) peuvent être obtenus par formylation des produits de formule générale:

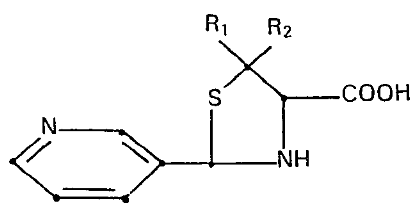

(VIII)

La formylation peut être avantageusement effectuée par action de l'acide formique dans l'anhydride acétique à une température comprise entre 10°C et 25°C.

Les produits de formule générale (VIII) peuvent être préparés selon la méthode de A. BANASHEK et M.I. SHCHUKINA, J. Gen. Chem. U.S.S.R. 31, 1374 (1961); Chem. Abstr. 55, 24739 h (1961).

Selon l'invention, les produits de formule générale (I) dans laquelle $R_1$ et $R_2$ sont définis comme précédemment et R représente un radical de formule générale (II) dans laquelle $R_3$ et $R_4$ forment ensemble un cycle imidazolyle peuvent être préparés par action du N,N'-carbonyldiimidazole sur un acide de formule générale:

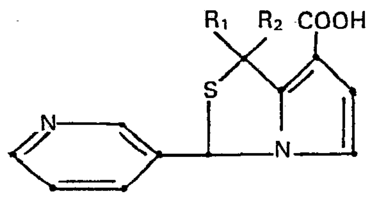

(IX)

dans laquelle $R_1$ et $R_2$ sont définis comme précédemment.

On opère généralement dans un solvent organique inerte tel que le tétrahydrofuranne ou le diméthylformamide à une température voisine de 20°C.

Les produits de formule générale (IX) peuvent être préparés par hydrolyse des nitriles de formule générale (V) par toute méthode connue de l'homme du métier pour transformer un nitrile en acide sans toucher au reste de la molécule, notamment par chauffage en milieu alcalin dans un alcool à haut point d'ébullition tel que l'éthylèneglycol, à une température comprise entre 100°C et le reflux du mélange réactionnel.

Selon l'invention, les produits de formule générale (I) dans laquelle $R_1$ et $R_2$ sont définis comme précédemment et R représente un radical de formule générale (II) dans laquelle $R_3$ et $R_4$ sont définis comme en b) à l'exception pour $R_3$ et $R_4$ de former ensemble un cycle imidazolyle peuvent être préparés par action de l'ammoniac ou d'un produit de formule générale:

$$HN\begin{matrix}R_3\\R_4\end{matrix} \qquad (X)$$

dans laquelle $R_3$ et $R_4$ sont définis comme précédemment en b) à l'exception pour $R_3$ et $R_4$ de former ensemble un cycle imidazolyle, sur un acide de formule générale (IX).

Lorsque $R_3$ ou $R_4$ représente un radical alcoyle contenant 1 à 5 atomes de carbone substitué par un radical amino, alcoylamino ou pipérazinyle ou bien que $R_3$ et $R_4$ forment ensemble avec l'atome d'azote auquel ils sont liés un hétérocycle à 5 ou 6 chaînons contenant un autre atome d'azote éventuellement substitué par un radical aminoalcoyle, les fonctions amines correspondantes doivent être préalablement protégées avant condensation sur l'acide de formule générale (IX).

Le blocage et le déblocage subséquent peuvent s'effectuer par toute méthode connue de l'homme du métier pour protéger une fonction amine primaire ou secondaire, par exemple sous forme de trifluoroacétamide, le déblocage étant réalisé à l'aide de méthanol ammoniacal.

Il est particulièrement avantageux d'utiliser l'acide de formule générale (IX) sous une forme activée par exemple:

α) sous forme de chlorure d'acide; dans ce cas, on opère dans un solvant halogéné tel que le chloroforme, le chlorure de méthylène ou le dichloro-1,2 éthane, ou un éther tel que le dioxanne, à une température comprise entre 20°C et la température de reflux du mélange réactionnel, ou

β) sous forme d'anhydride mixte, obtenue par action d'un chloroformiate d'alcoyle sur l'acide de formule générale (IX); dans ce cas on opère dans un solvant tel que l'éther ou le tétrahydrofuranne ou encore dans le diméthylformamide à une température comprise entre 20°C et le reflux du mélange réactionnel,

γ) sous forme d'imidazolide, c'est-à-dire un produit de formule générale (I) dans laquelle $R_1$ et $R_2$ sont définis comme précédemment et R représente un radical de formule générale (II) dans laquelle $R_3$ et $R_4$ forment ensemble un cycle imidazolyle; dans ce cas, la réaction s'effectue dans un solvant organique tel que le tétrahydrofuranne ou le diméthylformamide ou un mélange de ces solvants à une température comprise entre 20°C et le reflux du mélange réactionnel.

Selon l'invention, les produits de formule générale (I) dans laquelle $R_1$ et $R_2$ sont définis comme précédemment et R représente un radical de formule générale (II) dans laquelle $R_3$ et $R_4$ représentent chacun un atome d'hydrogène peuvent également être préparés par hydrolyse d'un nitrile de formule générale (V). L'hydrolyse peut être effectuée par tout moyen connu de l'homme du métier pour transformer un nitrile en amide sans toucher au reste de la molécule, notamment par chauffage en milieu alcalin dans un solvant organique tel que le tert-butanol.

Selon l'invention les produits de formule générale (I) dans laquelle $R_1$ et $R_2$ sont définis comme précédemment et R représente un radical de formule générale (II) dans laquelle $R_3$ et $R_4$ représentent chacun un atome d'hydrogène peuvent également être préparés par action de la chloro-2 acrylamide de formule:

$$CH_2 = C\begin{matrix}Cl\\CONH_2\end{matrix} \qquad (XI)$$

sur un acide de formule générale (VII) dans laquelle $R_1$ et $R_2$ sont définis comme précédemment.

La réaction s'effectue généralement dans l'anhydride acétique par chauffage à une température comprise entre 80°C et 130°C.

Selon l'invention les produits de formule générale (I) dans laquelle $R_1$ et $R_2$ sont définis comme précédemment, et R représente un radical acétyle peuvent être préparés par action du dérivé éthoxymagnésien du malonate d'éthyle sur un produit de formule générale:

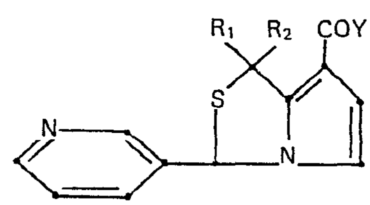

(XII)

dans laquelle $R_1$ et $R_2$ sont définis comme précédemment et Y représente un atome d'halogène puis hydrolyse et décarboxylation du produit obtenu.

On opère généralement dans un solvant organique tel qu'un éther ou un alcool ou un mélange de ces solvants, en présence d'un accepteur d'acide tel que la triéthylamine à une température comprise entre 10°C et la température de reflux du mélange réactionnel, l'hydrolyse et la décarboxylation s'effectuant selon les méthodes connues de l'homme du métier.

Les produits de formule générale (XII) peuvent être préparés à partir des acides de formule générale (IX) par toute méthode connue de l'homme du métier pour passer d'un acide à un halogénure d'acide.

Selon l'invention, les produits de formule générale (I) dans laquelle $R_1$ et $R_2$ sont définis comme précédemment, et R représente un radical alcoylcarbonyle peuvent être préparés par action d'un dérivé organomagnésien de formule générale:

$$R''' \; Mg \; X \qquad (XIII)$$

dans laquelle R''' représente un radical alcoyle et X représente un atome d'halogène sur un nitrile de formule générale (V), suivie d'une hydrolyse.

On opère par toute méthode connue de l'homme du métier pour obtenir une cétone à partir d'un nitrile et d'un dérivé organomagnésien sans toucher au reste de la molécule.

Selon l'invention, les produits de formule générale (I) dans laquelle $R_1$ et $R_2$ sont définis comme précédemment et R représente un radical de formule générale (IV) défini comme précédemment peuvent être préparés par action d'une hydrazine de formule générale:

$$H_2N\text{-}N{<}^{R'}_{R''} \qquad (XIV)$$

dans laquelle R' et R'' représentent des radicaux alcoyle identiques ou différents sur un produit de formule générale:

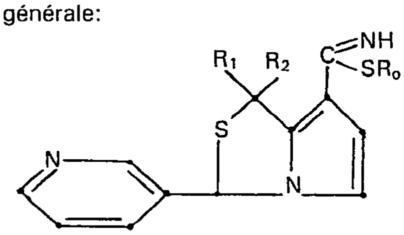

(XV)

dans laquelle $R_o$ représente un radical alcoyle et $R_1$ et $R_2$ sont définis comme précédemment.

On opère généralement dans un solvant organique tel que l'éthanol à une température comprise entre 20°C et 80°C.

Les produits de formule générale (XV) peuvent être obtenus par action d'un halogénure d'alcoyle de formule générale:

$$R_o - Z \qquad (XVI)$$

dans laquelle $R_o$ représente un radical alcoyle et Z représente un atome d'halogène, de préférence un atome d'iode, sur un produit de formule générale:

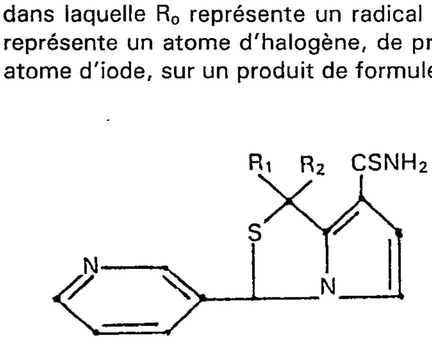

(XVII)

dans laquelle $R_1$ et $R_2$ sont définis comme précédemment.

On opère généralement dans un solvant organique tel que l'acétone ou le diméthylformamide ou un mélange de ces solvants à une température comprise entre 0°C et 50°C.

Les produits de formule générale (XVII) peuvent être préparés par thionation d'un produit de formule générale (I) dans laquelle $R_1$ et $R_2$ sont définis comme précédemment et R représente un radical de formule générale (II) dans laquelle $R_3$ et $R_4$ représentent un atome d'hydrogène.

La réaction s'effectue généralement au moyen d'un réactif de thionation par exemple au moyen du réactif de LAWESSON [bis(méthoxy-4 phényl)-2,4-dithioxo-2,4 dithia-1,3 diphospha-2,4 étanne] dans un solvant organique tel que le toluène à une température voisine de 50°C ou le diméthoxy-1,2 éthane ou l'hexaméthylphosphoramide à une température voisine de 20°C, ou bien au moyen du pentasulfure de phosphore dans un solvant organique tel que le toluène ou le dioxanne ou mieux dans un solvant tel que la pyridine en présence d'un courant d'hydrogène sulfuré.

Selon l'invention, les produits de formule générale (I) dans laquelle $R_1$ et $R_2$ sont définis comme précédemment et R représente un radical de formule (III) peuvent être préparés par action de l'hydroxylamine sur un produit de formule générale (XVII).

On emploie généralement le chlorhydrate d'hydroxylamine et on opère dans un solvant organique tel que la pyridine en présence de chlorure mercurique, à une température voisine de 20°C.

Les nouveaux produits de formule générale (I) peuvent être purifiés par les méthodes connues habituelles, par exemple cristallisation, chromatographie ou extractions successives en milieu acide et basique.

Les nouveaux produits de formule générale (I) peuvent être transformés en sel d'addition avec les acides par action d'un acide dans un solvant organique tel qu'un alcool, une cétone, un éther ou un solvant chloré. Le sel formé précipite, éventuellement après concentration de sa solution; il est séparé par filtration ou décantation.

Les nouveaux produits de formule générale (I) dans laquelle R représente un radical de formule générale (II) dans laquelle $R_3$ représente un atome d'hydrogène et $R_4$ représente un radical alcoyle contenant 1 à 5 atomes de carbone substitué par un radical carboxy peuvent être transformés en sels métalliques ou en sels d'addition avec les bases azotées par toute méthode connue de l'homme du métier pour effectuer cette transformation sans toucher au reste de la molécule.

Les nouveaux produits selon l'invention ainsi que leurs sels d'addition représentent des propriétés pharmacologiques intéressantes associées à une faible toxicité. Ils se sont montrés actifs à des concentrations inférieures à 50 mg/l dans le test de mesure de l'activité inhibitrice in vitro vis-à-vis de l'agrégation plaquettaire induite par l'O-octadécyl-1 O-acétyl-2 ns-glycérophosphorylcholine-3 (P.A.F.-Acéther) selon la technique de G.V.R. BORN et coll. J. Physiol. 168, 178 (1963).

Leur dose toxique (exprimée par la $DL_{50}$) chez la souris est généralement comprise entre 300 et 900 mg/kg par voie orale.

Sont particulièremennt intéressants les produits de formule générale (I) dans laquelle $R_1$ et $R_2$ représentent tous les deux un atome d'hydrogène ou un radical alcoyle et

a) soit R représente un radical cyano ou alcoylcarbonyle

b) soit R représente un radical de formule générale (II) dans laquelle:

— ou bien $R_3$ représente un atome d'hydrogène et $R_4$ représente un radical amino,

— ou bien $R_3$ et $R_4$, identiques ou différents, représentent un atome d'hydrogène ou un radical alcoyle ou phényle éventuellement substitué par un atome d'halogène ou un radical alcoyle, alcoyloxy ou diméthylamino,

— ou bien $R_3$ représente un atome d'hydrogène et $R_4$ représente un radical pyridyle ou alcoyle contenant 1 à 5 atomes de carbone substitué par un radical carboxy, dialcoylamino, pipéridino, pipérazinyle-1 (substitué en position 4 par un radical pyridyle), phényle ou imidazolyle,

— ou bien $R_3$ et $R_4$ forment ensemble un cycle imidazolyle ou pipérazinyle substitué en position 4 par un radical hydroxyalcoyle, pyridyle, pyrimidyle, phényle (éventuellement substitué par un radical alcoyloxy) ou benzyle

c) soit R représente un radical de formule générale (III) ou (IV) dans laquelle R' et R'', identiques, représentent un radical alcoyle.

Sont plus particulièrement intéressants les produits de formule générale (I) dans laquelle $R_1$ et $R_2$ représentent un atome d'hydrogène et R représente un radical de formule générale (II) dans laquelle

— ou bien $R_3$ et $R_4$, identiques ou différents, représentent un atome d'hydrogène ou un radical alcoyle ou phényle,

— ou bien $R_3$ représente un atome d'hydrogène et $R_4$ représente un radical pyridyle ou alcoyle substitué par un radical phényle ou imidazolyle,

— ou bien $R_3$ et $R_4$ forment ensemble un cycle pipérazinyle substitué en position 4 par un radical pyridyle, phényle ou benzyle.

Sont d'un intérêt particulier, les produits suivants:

-N-Pyridyl-2 (pyridyl-3)-3 1H,3H-pyrrolo [1,2-c] thiazolecarboxamide-7

-N-Phényl (pyridyl-3)-3 1H,3H-pyrrolo [1,2-c] thiazolecarboxamide-7,

-(Pyridyl-3)-3 1H,3H-pyrrolo [1,2-c] thiazolecarboxamide-7,

-N-Pyridyl-3 (pyridyl-3)-3 1H,3H-pyrrolo [1,2-c] thiazolecarboxamide-7,

-N-Méthyl (pyridyl-3)-3 1H,3H-pyrrolo [1,2-c] thiazolecarboxamide-7'

-N-Butyl (pyridyl-3)-3 1H,3H-pyrrolo [1,2-c] thiazolecarboxamide-7,

-N-Benzyl (pyridyl-3)-3 1H,3H-pyrrolo [1,2-c] thiazolecarboxamide-7,

-N-[(Imidazolyl-4)-2 éthyl] (pyridyl-3)-3 1H,3H-pyrrolo [1,2-c] thiazolecarboxamide-7,

-(Phényl-4 pipérazinyl-1) carbonyl-7 (pyridyl-3)-3 1H,3H-pyrrolo [1,2-c] thiazole,

-[(Pyridyl-2)-4 pipérazinyl-1] carbonyl-7 (pyridyl-3)-3 1H,3H-pyrrolo [1,2-c] thiazole,

-(Benzyl-4 pipérazinyl-1) carbonyl-7 (pyridyl-3)-3 1H,3H-pyrrolo [1,2-c] thiazole.

Pour l'emploi thérapeutique, il peut être fait usage des nouveaux produits de formule générale (I), tels quels ou, le cas échéant, à l'état de sels pharmaceutiquement acceptables, c'est-à-dire non toxiques aux doses d'utilisation.

Comme sels pharmaceutiquement acceptables peuvent être cités les sels d'addition avec les acides minéraux tels que chlorhydrates, sulfates, nitrates, phosphates ou organiques tels que acétates, propionates, succinates, benzoates, fumarates, maléates, méthanesulfonates, iséthionates, théophillineacétates, salicylates, phénolphtalinates, méthylène-bis-β-oxynaphtoates ou des dérives de substitution de ces composés.

Les exemples suivants, donnés à titre non limitatif, montrent comment l'invention peut être mise en pratique.

*Exemple 1*

Un mélange de 249,4 g d'acide formyl-3 (pyridyl-3)-2 thiazolidinecarboxylique-4, de 457 g de chloro-2 acrylonitrile et de 0,2 g d'hydroquinone dans 1760 $cm^3$ d'anhydride acétique est chauffé à une température comprise entre 110°C et 117°C pentant 70 minutes. Le solvant est ensuite évaporé sous pression réduite (20 mm de mercure; 2,7 kPa) à une température comprise entre 50°C et 80°C. Le résidu est repris par 400 $cm^3$ d'eau distillée; la suspension obtenue est amenée à un pH voisin de 10 par addition d'une solution aqueuse de soude 5N puis extraite 4 fois par 2500 $cm^3$ au total de chlorure de méthylène. Les extraits organiques sont réunis, lavés 3 fois par 1500 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 40°C. Le résidue obtenu est dissous dans un mélange de 250 $cm^3$ d'acétate d'éthyle et de 500 $cm^3$ d'une solution aqueuse d'acide chlorhydrique 1,8N. La phase organique est séparée par décantation et extraite 2 fois par 200 $cm^3$ au total d'eau distillée. Les extraits aqueux sont réunis, lavés 5 fois par 500 $cm^3$ au total d'acétate d'éthyle, additionnés de 0,5 g de noir décolorant et filtrés; le filtrat est amené à un pH voisin de 10 par addition d'une solution aqueuse de soude 10N à une température voisine de 4°C puis extrait 3 fois avec 650 $cm^3$ au total d'acétate d'éthyle. Les extraits organiques sont réunis, lavés 3 fois par 450 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 40°C. On obtient ainsi 71,2 g de produit brut. Ce produit est dissous dans 150 $cm^3$ de propanol-2 bouillant et la solution obtenue est additionnée de 0,5 g de noir décolorant puis filtrée à chaud; le filtrat est refroidi à une température voisine de 4°C pentant 1 heure. Les cristaux apparus sont séparés par filtration, lavés 3 fois par 30 $cm^3$ au total de propanol-2 refroidi à une température voisine de 4°C, 3 fois par

60 $cm^3$ au total d'oxyde d'isopropyle puis séchés sous pression réduite (20 mm de mercure; 2,7 kPa) à une température voisine de 20°C en présence de potasse en pastilles. On obtient ainsi 44 g de (pyridyl-3)-3 1H,3H-pyrrolo [1,2-c] thiazolecarbonitrile-7 sous forme de cristaux de couleur ocre fondant à 117°C.

L'acide formyl-3 (pyridyl-3)-2 thiazolidinecarboxylique-4 peut être obtenu de la façon suivante:

A 1200 $cm^3$ d'acide formique, on ajoute 250 g d'acide (pyridyl-3)-2 thiazolidinecarboxylique-4 en maintenant la température du milieu réactionnel inférieure à 25°C. A la solution ainsi obtenue on ajoute, en 1 heure, 875 g d'anhydride acétique en maintenant la température entre 10°C et 18°C.

Après 20 heures d'agitation, à une température voisine de 20°C, on évapore le solvant sous pression réduite (20 mm de mercure; 2,7 kPa) à une température voisine de 60°C, reprend le résidu avec 1000 $cm^3$ d'éthanol, chauffe à ébullition pendant 5 minutes puis refroidit à une température voisine de 4°C pentant 1 heure; les cristaux apparus sont séparés par filtration, lavés 3 fois par 600 $cm^3$ au total d'éthanol puis 3 fois par 300 $cm^3$ au total d'éther diéthylique et séchés sous pression réduite (20 mm de mercure; 2,7 kPa) à une température voisine de 20°C en présence de potasse en pastilles. On obtient ainsi 234,5 g d'acide formyl-3 (pyridyl-3)-2 thiazolidinecarboxylique-4 sous forme de cristaux crème fondant à 214°C.

L'acide (pyridyl-3)-2 thiazolidinecarboxylique-4 peut être préparé selon A. BANASHEK et M.I. SHCHUKINA, J. Gen. Chem. U.S.S.R., 31, 1374 (1961); Chem. Abstr. 55, 24739 h (1961).

*Exemple 2*

A une solution de 10,2 g d'acide (pyridyl-3)-3 1H,3H-pyrrolo [1,2-c] thiazolecarboxylique-7 et de 10,1 g de N,N'-carbonyldiimidazole dans 150 $cm^3$ de tétrahydrofuranne anhydre agitée sous azote sec à une température voisine de 20°C pendant 1 heure et 20 minutes, on ajoute une solution de 9,5 g de diéthylamino-2 éthylamine dans 50 $cm^3$ de tétrahydrofuranne anhydre en 10 minutes à une température voisine de 25°C. Après une heure d'agitation à une température voisine de 20°C, on évapore le solvant sous pression réduite (20 mm de mercure; 2,7 kPa) à une température voisine de 40°C. Le résidu obtenu est repris par 700 $cm^3$ d'eau distillée et le mélange est extrait 5 fois par 750 $cm^3$ au total d'acétate d'éthyle. Les extraits organiques sont réunis, lavés 5 fois avec 500 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 40°C. On obtient ainsi 12,4 g de produit brut. Ce produit, réuni avec 2 g de produit préparé de la même manière dans une autre opération antérieure, est dissous dans 100 $cm^3$ d'un mélange bouillant de cyclohexane et d'acétate d'éthyle (50-50 en volumes). La solution obtenue est additionnée de 0,5 g de noir décolorant puis filtrée à chaud; le filtrat est refroidi à une température voisine de 4°C pendant 2 heures. Les cristaux apparus sont séparés par filtration, lavés 3 fois par 45 $cm^3$ au total d'oxyde d'isopropyle et séchés sous pression réduite (20 mm de mercure; 2,7 kPa) à une température voisine de 20°C en présence de potasse en pastilles. On obtient ainsi 9,8 g de N-(diéthylamino-2 éthyl) (pyridyl-3)-3 1H,3H-pyrrolo [1,2-c] thiazolecarboxamide-7 sous forme de cristaux blancs fondant à 129°C.

L'acide (pyridyl-3)-3 1H,3H-pyrrolo [1,2-c] thiazolecarboxamide-7 peut être obtenu de la façon suivante:

48 g de (pyridyl-3)-3 1H,3H-pyrrolo [1,2-c] thiazolecarbonitrile-7 sont ajoutés à une solution de 41,7 g de potasse en pastilles dans 400 $cm^3$ d'éthylèneglycol. Le mélange réactionnel est chauffé à une température voisine de 150°C pendant 6 heures et 30 minutes. Après 16 heures d'agitation à une température voisine de 20°C, on évapore le solvent sous pression réduite (5 mm de mercure; 0,7 kPa) à une température voisine de 100°C. Le rédisu est dissous dans 380 $cm^3$ d'eau distillée. La solution obtenue est additionnée de 0,5 g de noir décolorant, filtrée et amenée à un pH voisin de 4 par addition d'une solution aqueuse concentrée d'acide chlorhydrique en maintenant la température voisine de 20°C. Les cristaux apparus sont séparés par filtration, lavés 3 fois avec 600 $cm^3$ au total d'eau distillée puis séchés sous pression réduite (20 mm de mercure; 2,7 kPa) à une température voisine de 20°C en présence de potasse en pastilles. On obtient ainsi 49,5 g de produit brut fondant à 177°C. Ce produit est dissous dans 840 $cm^3$ d'éthanol bouillant. La solution obtenue est additionnée de 0,5 g de noir décolorant puis filtrée à chaud; le filtrat est refroidi à une température voisine de 4°C pendant 1 heure. Les cristaux apparus sont séparés par filtration, lavés 3 fois aves 45 $cm^3$ au total d'éthanol refroidi à une température voisine de 4°C puis 3 fois avec 90 $cm^3$ au total d'éther diéthylique et séchés sous pression réduite (20 mm de mercure; 2,7 kPa) à une température voisine de 20°C en présence de potasse en pastilles. On obtient ainsi 36,6 g d'acide (pyridyl-3)-3 1H,3H-pyrrolo [1,2-c] thiazolecarboxylique-7 sous forme de cristaux crème fondant à 178°C.

*Exemple 3*

On chauffe sous agitation pendant 3 heures et 15 minutes à une température voisine de 85°C un mélange de 13,6 g de (pyridyl-3)-3 1H,3H-pyrrolo [1,2--c] thiazolecarbonitrile-7 et de 21 g de potasse en poudre dans 150 $cm^3$ d'alcool tert-butylique. On évapore ensuite le solvant sous pression réduite (20 mm de mercure; 2,7 kPa) à une température voisine de 40°C puis remet le résidu en suspension dans 500 $cm^3$ d'eau distillée et agite pendant 5 minutes à une température voisine de 20°C; les cristaux apparus sont séparés par filtration, lavés 5 fois par 500 $cm^3$ au total d'eau distillée, 3 fois par 60 $cm^3$ au total d'éthanol, puis 3 fois par 60 $cm^3$ au total d'éther diéthylique et séchés sous pression réduite (20 mm de mercure; 2,7 kPa) à une température voisine de 20°C en présence de potasse en pastilles. On obtient ainsi 12,1 g de produit brut fondant à 208°C. Ce produit, réuni avec 2,2 g de priduit préparé de la même manière dans une autre opération antérieure est dissous dans 800 $cm^3$ d'éthanol bouillant. La solution

obtenue est additionnée de 0,5 g de noir décolorant puis filtrée à chaud. Le filtrat est refroidi pendant 1 heure à une température voisine de 4°C; les cristaux apparus sont séparés par filtration, lavés 3 fois par 30 $cm^3$ au total d'éthanol refroidi à une température voisine de 4°C et séchés sous pression réduite (20 mm de mercure; 2,7 kPa) à une température voisine de 20°C en présence de potasse en pastilles. On obtient ainsi 10,7 g de (pyridyl-3)-3 1H,3H-pyrrolo [1,2-c] thiazolecarboxamide-7 sous forme de cristaux blancs fondant à 210°C.

Le (pyridyl-3)-3 1H,3H-pyrrolo [1,2-c] thiazolecarbonitrile-7 est préparé comme à l'exemple 1.

*Exemple 4*

On agite sous azote sec à une température voisine de 20°C pendant 1 heure une solution de 6,16 g d'acide (pyridyl-3)-3 1H,3H-pyrrolo [1,2-c] thiazolecarboxylique-7 et de 4,45 g de N,N'-carbonyldiimidazole dans 50 $cm^3$ de tétrahydrofuranne anhydre. A la solution ainsi obtenue, on ajoute en 20 minutes à une température voisine de 25°C une solution de 3,87 g de N-(Amino-2 éthyl) pipéridine dans 10 $cm^3$ de tétrahydrofuranne anhydre. Après 16 heures d'agitation à une température voisine de 20°C on ajoute 25 $cm^3$ d'eau distillée au milieu réactionnel et évapore le solvant sous pression réduite (20 mm de mercure; 2,7 kPa) à une température voisine de 40°C. Le rédisu est dissous dans 300 $cm^3$ d'acétate d'éthyle et la solution ainsi obtenue est lavée 5 fois par 400 $cm^3$ au total d'eau distillée, séchée sur du sulfate de magnésium anhydre, additionnée de 0,2 g de noir décolorant, filtrée et concentrée à sec sous pression réduite (20 mm de mercure; 2,7 kPa) à une température voisine de 40°C. Le résidu est dissous dans 100 $cm^3$ d'une solution aqueuse d'acide chlorhydrique 0,4N et la solution obtenue est extraite 3 fois par 120 $cm^3$ au total de chlorure de méthylène puis additionnée de 0,2 g de noir décolorant et filtrée. Le filtrat est amené à un pH voisin de 10 par addition d'une solution aqueuse de soude 10N et est extrait 5 fois par 400 $cm^3$ au total d'acétate d'éthyle. Les extraits organiques sont réunis, lavés 5 fois par 400 $cm^3$ au total d'eau distillée, séchés sur du sulfate de magnésium anhydre, additionnée de 0,2 g de noir décolorant, filtrés et concentrés à sec sous pression réduite (20 mm de mercure; 2,7 kPa) à une température voisine de 40°C. On obtient ainsi 7,6 g de produit brut. Ce produit est dissous dans 80 $cm^3$ d'acétonitrile bouillant et la solution est additionnée de 0,2 g de noir décolorant puis filtrée à chaud; le filtrat est refoidi à une température voisine de 4°C pendant 16 heures. Les cristaux apparus sont séparés par filtration, lavés 3 fois par 20 $cm^3$ au total d'acétonitrile refoidi à une température voisine de 4°C et séchés sous pression réduite (20 mm de mercure; 2,7 kPa) à une température voisine de 20°C en présence de potasse en pastilles. On obtient ainsi 5,9 g de N-(pipéridino-2 éthyl) (pyridyl-3)-3 1H,3H-pyrrolo [1,2-c] thiazolecarboxamide-7 sous forme de cristaux blancs fondant à 150°C.

L'acide (pyridyl-3)-3 1H,3H-pyrrolo [1,2-c] thiazolecarboxylique-7 est preparé comme à l'exemple 2.

*Exemple 5*

On agite sous azote sec à une température voisine de 20°C pendant 1 heure et 20 minutes une solution de 7,6 g d'acide (pyridyl-3)-3 1H,3H-pyrrolo [1,2-c] thiazolecarboxylique-7 et de 6,9 g de N,N'-carbonyldiimidazole dans 25 $cm^3$ de tétrahydrofuranne anhydre. A la solution ainsi obtenue on ajoute en 10 minutes à une température voisine de 25°C 5,4 g de diméthylamino-2 éthylamine dilués dans 30 $cm^3$ de tétrahydrofuranne anhydre. Après 16 heures d'agitation à une température voisine de 20°C, on ajoute 20 $cm^3$ d'eau distillée au mélange réactionnel et évapore le solvant sous pression réduite (20 mm de mercure; 2,7 kPa) à une température voisine de 40°C. Le résidu est dissous dans 350 $cm^3$ d'acétate d'éthyle et la solution ainsi obtenue est lavée 5 fois par 400 $cm^3$ au total d'eau distillée, séchée sur sulfate de magnésium anhydre, additionnée de 0,2 g de noir décolorant, filtrée et concentrée à sec sous pression réduite (20 mm de mercure; 2,7 kPa) à une température voisine de 40°C. On obtient ainsi 6,2 g de produit brut. Ce produit est chomatographié sur une colonne de 2,5 cm de diamètre contenant 60 g de silice (0,063-0,2 mm). On élue par un mélange d'acétate d'éthyle et de méthanol (50/50 en volumes) en recueillant des fractions de 100 $cm^3$. La première fraction est éliminée; les 9 fractions suivantes sont réunies et concentrées à sec sous pression réduite (20 mm de mercure; 2,7 kPa) à une température voisine de 40°C. On obtient ainsi 5,6 g de produit que l'on redissout dans 60 $cm^3$ d'acétonitrile bouillant. La solution obtenue est additionnée de 0,2 g de noir décolorant et filtrée à chaud; le filtrat est refroidi à une température voisine de 4°C pendant 1 heure. Les cristaux apparus sont séparés par filtration, lavés 3 fois par 10 $cm^3$ au total d'acétonitrile refroidi à une température voisine de 4°C et séchés sous pression réduite (20 mm de mercure; 2,7 kPa) à une température voisine de 20°C en présence de potasse en pastilles. On obtient ainsi 2,3 g de N-diméthylamino-2 éthyl) (pyridyl-3)-3 1H,3H-pyrrolo [1,2-c] thiazolecarboxamide-7 sous forme de cristaux crème fondant à 150°C.

L'acide (pyridyl-3)-3 1H,3H-pyrrolo [1,2-c] thiazolecarboxylique-7 est préparé comme à l'exemple 2.

*Exemple 6*

On agite sous azote sec à une température voisine de 20°C pendant 2 heures et 20 minutes une solution de 7,4 g d'acide (pyridyl-3)-3 1H,3H-pyrrolo [1,2-c] thiazolecarboxylique-7 et de 5,35 g de N,N'-carbonyldiimidazole dans 50 $cm^3$ de tétrahydrofuranne anhydre. La solution obtenue est saturée par un courant de monométhylamine en maintenant la température du mélange réactionnel voisine de 20°C pendant 1 heure et 30 minutes. Après 16 heures d'agitation à une température voisine de 20°C, le mélange réactionnel est additionné de 500 $cm^3$ d'eau distillée et refroidi pendant 30 minutes à une température voisine de 4°C; les cristaux apparus sont séparés par filtration, lavés 3 fois par 150 $cm^3$ au total d'eau distillée, séchés sous pression réduite (20 mm de mercure; 2,7 kPa) à une température voisine de 20°C en présence de potasse en pastilles. On obtient ainsi 5,5 g de produit brut fondant à 168°C.

Ce produit est dissous dans 65 $cm^3$ d'acétonitrile bouillant. La solution est additionnée de 0,5 g de noir décolorant et filtrée à chaud; le filtrat est refroidi à une température voisine de 4°C pendant 30 minutes. Les cristaux apparus sont séparés par filtration, lavés 3 fois par 10 $cm^3$ au total d'acétonitrile refroidi à une température voisine de 4°C, séchés sous pression réduite (20 mm de mercure; 2,7 kPa) à une température voisine de 20°C en présence de potasse en pastilles. On obtient ainsi 4,3 g de N-méthyl (pyridyl-3)-3 1H,3H-pyrrolo [1,2-c] thiazolecarboxamide-7 sous forme de cristaux blancs fondant à 170°C.

L'acide (pyridyl-3)-3 1H,3H-pyrrolo [1,2-c] thiazolecarboxylique-7 est préparé comme à l'exemple 2.

*Exemple 7*

On agite sous azote sec à une température voisine de 20°C pendant 4 heures une solution de 7,4 g d'acide (pyridyl-3)-3 1H,3H-pyrrolo [1,2-c] thiazolecarboxylique-7 et de 7,3 g de N,N'-carbonyldiimidazole dans 50 $cm^3$ de tétrahydrofuranne anhydre. La solution obtenue est saturée pendant 2 heures à une température voisine de 10°C par un courant de diméthylamine anhydre. Après 16 heures d'agitation à une température voisine de 20°C, le milieu réactionnel est additionné de 30 $cm^3$ d'eau distillée à une température comprise entre 21°C et 29°C puis le solvant est évaporé sous pression réduite (20 mm de mercure; 2,7 kPa) à une température voisine de 40°C. Le résidu et dissous dans 250 $cm^3$ de chlorure de méthylène et la solution obtenue est lavée 5 fois par 750 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 40°C. On obtient ainsi 7,5 g de produit brut. Ce produit est chromatographié sur une colonne de 5 cm de diamètre contenant 400 g de silice (0,040-0,063 mm). On élue par un mélange de chlorure de méthylene et de méthanol (95/5 en volumes) sous une pression de 0,5 bar (51 kPa) en recueillant des fractions de 75 $cm^3$. Les 4 premières fractions sont éliminées; les 10 fractions suivantes sont réunies et concentrées à sec sous pression réduite (20 mm de mercure; 2,7 kPa) à une température voisine de 40°C. On obtient ainsi 6,8 g de produit. Ce produit est dissous dans 30 $cm^3$ d'acétonitrile bouillant. La solution est additionnée de 0,5 g de noir décolorant et filtrée à chaud; le filtrat est refroidi à une température voisine de 4°C pendant 1 heure. Les cristaux apparus sont séparés par filtration, lavés 3 fois par 10 $cm^3$ au total d'acétonitrile refroidi à une température voisine de 4°C, séchés sous pression réduite (20 mm de mercure; 2,7 kPa) à une température voisine de 20°C en présence de potasse en pastilles. On obtient ainsi 4,4 g de N,N-diméthyl (pyridyl-3)-3 1H,3H-pyrrolo [1,2-c] thiazolecarboxamide-7 sous forme de cristaux crème fondant à 129°C.

L'acide (pyridyl-3)-3 1H,3H-pyrrolo [1,2-c] thiazolecarboxylique-7 est préparé comme à l'exemple 2.

*Exemple 8*

On chauffe sous agitation pendant 7 heures et 20 minutes à une température voisine de 84°C un mélange de 9,9 g de diméthyl-1,1 (pyridyl-3)-3 1H,3H-pyrrolo [1,2-c] thiazolecarbonitrile-7 et de 13,6 g de potasse en poudre dans 140 $cm^3$ d'alcool tert-butylique. Après 16 heures d'agitation à une température voisine de 20°C, le mélange réactionnel est chauffé sous agitation pendant 3 heures et 30 minutes supplémentaires à une température voisine de 85°C. Le solvant est évaporé sous pression réduite (20 mm de mercure; 2,7 kPa) à une température voisine de 40°C. Le résidu est mis en suspension dans 300 $cm^3$ d'eau distillée et agité pendant 15 minutes à une température voisine de 4°C; les cristaux apparus sont séparés par filtration, lavés 3 fois par 300 $cm^3$ au total d'eau distillée et séchés sous pression réduite (20 mm de mercure; 2,7 kPa) à une température voisine de 20°C en présence de potasse en pastilles. On obtient ainsi 10,5 g de produit brut. Ce produit est dissous dans 65 $cm^3$ d'acétate d'éthyle bouillant et la solution obtenue est additionnée de 0,5 g de noir décolorant et filtrée à chaud; le filtrat est refroidi à une température voisine de 4°C pendant 1 heure. Les cristaux apparus sont séparés par filtration, lavés 3 fois par 10 $cm^3$ au total d'acétate d'éthyle refroidi à une température voisine de 4°C et séchés sous pression réduite (20 mm de mercure; 2,7 kPa) à une température voisine de 20°C en présence de potasse en pastilles. On obtient ainsi 9 g de diméthyl-1,1 (pyridyl-3)-3 1H,3H-pyrrolo [1,2-c] thiazolecarboxamide-7 sous forme de cristaux blancs fondant à 140°C.

Le diméthyl-1,1 (pyridyl-3)-3 1H,3H-pyrrolo [1,2-c] thiazolecarbonitrile-7 peut être préparé de la façon suivante:

A une solution de 71,4 g d'acide diméthyl-5,5 formyl-3 (pyridyl-3)-2 thiazolidinecarboxylique-4 et de 0,5 g d'hydroquinone dans 450 $cm^3$ d'anhydride acétique on ajoute à une température voisine de 80°C 117 g de chloro-2 acrylonitrile, Après 1 heure et 30 minutes d'agitation à une température voisine de 110°C, le mélange réactionnel est concentré à sec sous pression réduite (20 mm de mercure; 2,7 kPa) à une température comprise entre 50°C et 80°C. Le résidu est repris par 200 $cm^3$ d'eau distillée refroidie à une température voisine de 0°C; la suspension obtenue est amenée à un pH voisin de 10 par addition d'une solution aqueuse de soude 2N puis extrait 3 fois par 800 $cm^3$ au total d'acétate d'éthyle. Les extraits organiques sont réunis, lavés 5 fois par 750 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 40°C. Le résidu est repris par 200 $cm^3$ d'une solution aqueuse d'acide chlorhydrique 2N et la suspension est extraite par 200 $cm^3$ d'acétate d'éthyle sous agitation pendant 10 minutes. La phase organique est séparée et réextraite 2 fois par 100 $cm^3$ au total d'une solution aqueuse d'acide chlorhydrique 2N. Les extraits aqueux sont réunis, additionnés de 0,5 g de noir décolorant, filtrés, refroidis à une température voisine de 4°C, amenés à un pH voisin de 10 par addition d'une solution aqueuse de soude 10N. On extrait alors 6 fois par 900 $cm^3$ au total d'acétate d'éthyle.

Les extraits organiques sont réunis, lavés 5 fois par 750 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 40°C. On obtient ainsi 29,4 g de produit brut. Ce produit est chromatographié sur une colonne de 4,5 cm de diamètre contenant 290 g de silice (0,063-0,2 mm) en recueillant des fractions de 500 $cm^3$. Les 4 premières fractions provenant de l'élution par un mélange de cyclohexane et d'acétate d'éthyle (80/20 en volumes) sont éliminées. La cinquième fraction provenant de l'élution par un mélange de cyclohexane et d'acétate d'éthyle (80/20 en volumes) ainsi que les 7 fractions suivantes provenant de l'élution par un mélange de cyclohexane et d'acétate d'éthyle (75/25 en volumes) et les 4 fractions suivantes provenant de l'élution par un mélange de cyclohexane et d'acétate d'éthyle (70/30 en volumes) sont réunies et concentrées à sec sous pression réduite (20 mm de mercure; 2,7 kPa) à une température voisine de 40°C. On obtient ainsi 25,8 g de produit cristallisé. Ce produit est mis en suspension dans 150 $cm^3$ d'oxyde d'isopropyle. Les cristaux sont séparés par filtration, lavés 3 fois par 150 $cm^3$ au total d'oxyde d'isopropyle et séchés sous pression réduite (20 mm de mercure; 2,7 kPa) à une température voisine de 20°C en présence de potasse en pastilles. On obtient ainsi 20 g de produit fondant à 111°C.

Ce produit est chromatographié sur une colonne de 6 cm de diamètre contenant 480 g de silice (0,04-0,063 mm). On élue par un mélange de cyclohexane et d'acétate d'éthyle (55/45 en volumes) sous une pression de 0,5 bar (51 kPa) en recueillant des fractions de 500 $cm^3$. Les 3 premières fractions sont éliminées, les 6 fractions suivantes sont réunies, concentrées à sec sous pression réduite (20 mm de mercure; 2,7 kPa) à une température voisine de 40°C. On obtient ainsi 19,8 g de diméthyl-1,1 (pyridyl-3)-3 1H,3H-pyrrolo [1,2-c] thiazolecarbonitrile-7 sous forme de cristaux blancs fondant à 112°C.

L'acide diméthyl-5,5 formyl-3 (pyridyl-3)-2 thiazolidinecarboxylique-4 peut être obtenu de la façon suivante:

A une solution de 91 g d'acide diméthyl-5,5 (pyridyl-3)-2 thiazolidinecarboxylique-4 dans 420 $cm^3$ d'acide formique on ajoute en 45 minutes à une température comprise entre 8°C et 15°C 302 g d'anhydride acétique. Après 16 heures d'agitation à une température voisine de 20°C, on ajoute 65 $cm^3$ d'eau distillée au milieu réactionnel et on évapore le solvant sous pression réduite (20 mm de mercure; 2,7 kPa) à une température voisine de 60°C. Le résidu est repris par 250 $cm^3$ d'éthanol et le solvant est évaporé sous pression réduite (20 mm de mercure; 2,7 kPa) à une température voisine de 60°C. On obtient ainsi 115 g de produit brut. Ce produit est repris par 250 $cm^3$ d'éthanol bouillant. Après refroidissement à une température voisine de 4°C, les cristaux sont séparés par filtration, lavés 3 fois par 60 $cm^3$ au total d'éthanol refroidi à une température voisine de 4°C puis 3 fois par 150 $cm^3$ au total d'éther diéthylique et séchés sous pression réduite (20 mm de mercure; 2,7 kPa) à une température voisine de 20°C en présence de potasse en pastilles. On obtient ainsi 71,6 g d'acide diméthyl-5,5 (pyridyl-3)-2 thiazolidinecarboxylique-4 sous forme de cristaux blancs fondant à 185°C.

L'acide diméthyl-5,5 (pyridyl-3)-2 thiazolidinecarboxylique-4 peut être obtenu de la façon suivante:

A une solution de 30 g de DL-pénicillamine dans un mélange de 860 $cm^3$ d'éthanol et de 320 $cm^3$ d'eau distillée chauffé à l'ébullition, on ajoute en 5 minutes à une température voisine de 73°C 34 g de nicotinaldéhyde et maintient le chauffage sous agitation à cette température pendant 2 heures et 15 minutes. Après refroidissement à une température voisine de 20°C et agitation pendant 16 heures, on évapore le solvant sous pression réduite (20 mm de mercure; 2,7 kPa) à une température voisine de 50°C. Le résidu est repris par 250 $cm^3$ d'éthanol et le solvant est évaporé sous pression réduite (20 mm de mercure; 2,7 kPa) à une température voisine de 50°C. Cette opération est répétée une fois puis le résidu finalement recueilli est dissous dans 250 $cm^3$ d'éthanol bouillant. La solution obtenue est refroidie à une température voisine de 4°C pendant 16 heures. Les cristaux apparus sont séparés par filtration, lavés 3 fois par 30 $cm^3$ au total d'éthanol refroidi à une température voisine de 4°C puis 3 fois par 45 $cm^3$ au total d'éther diéthylique et séchés sous pression réduite (20 mm de mercure; 2,7 kPa) à une température voisine de 20°C en présence de potasse en pastilles. On obtient ainsi 26,7 g de produit fondant à 163°C.

Les liqueurs-mères de cristallisation sont concentrées à sec sous pression réduite (20 mm de mercure; 2,7 kPa) à une température voisine de 50°C. On obtient ainsi 27 g d'une huile épaisse. Cette huile est dissoute dans 80 $cm^3$ d'acétonitrile bouillant. Après refroidissement à une température voisine de 4°C pendant 1 heure, les cristaux apparus sont séparés par filtration, lavés 3 fois par 30 $cm^3$ au total d'acétonitrile refroidi à une température voisine de 4°C et séchés sous pression réduite (20 mm de mercure; 2,7 kPa) à une température voisine de 20°C en présence de potasse en pastilles. On obtient ainsi 12,4 g de produit fondant à 160°C. Ce produit est réuni au lot provenant de la première cristallisation et est dissous dans 150 $cm^3$ d'éthanol bouillant. On ajoute 0,5 g de noir décolorant à la solution obtenue et on filtre à chaud. Le filtrat est refroidi à une température voisine de 4°C pendant 1 heure. Les cristaux apparus sont séparés par filtration, lavés 3 fois par 30 $cm^3$ au total d'éthanol refroidi à une température voisine de 4°C et séchés sous pression réduite (20 mm de mercure; 2,7 kPa) à une température voisine de 20°C en présence de potasse en pastilles. On obtient ainsi 22,8 g d'acide diméthyl-5,5 (pyridyl-3)-2 thiazolidinecarboxylique-4 sous forme de cristaux blancs fondant à 173°C.

*Exemple 9*

Une solution de 1,1 g d'acide (pyridyl-3)-3 1H,3H-pyrrolo [1,2-c] thiazolecarboxylique-7 et de 10,9 g de N,N'-carbonyldiimidazole dans 150 $cm^3$ de tétrahydrofuranne anhydre est agitée sous azote sec à une température voisine de 20°C pendant 2 heures.

A la solution obtenue, on ajoute en 1 heure à une température comprise entre 23°C et 27°C une solution de 7,3 g de butylamine dans 20 $cm^3$ de tétrahydrofuranne anhydre. La suspension obtenue est agitée pendant 72 heures à une température voisine de 25°C puis additionnée de 35 $cm^3$ d'eau distillée. Le solvant est évaporé sous pression réduite (20 mm de mercure; 2,7 kPa) à une température voisine de 50°C. Le solide obtenu est mis en suspension dans 150 $cm^3$ d'eau distillée et les cristaux sont séparés par filtration, lavés 5 fois par 150 $cm^3$ au total d'eau distillée et séchés sous pression réduite (20 mm de mercure; 2,7 kPa) à une température voisine de 20°C en présence de potasse en pastilles. On obtient ainsi 14,8 g de produit humide fondant à 159°C. Ce produit est dissous dans 65 $cm^3$ d'éthanol bouillant. La solution obtenue est additionnée de 0,5 g de noir décolorant et filtrée à chaud. Le filtrat est refroidi à une température voisine de 4°C pendant 16 heures. Les cristaux apparus sont séparés par filtration, lavés 3 fois par 20 $cm^3$ au total d'éthanol refroidi à une température voisine de 4°C et séchés sous pression réduite (20 mm de mercure; 2,7 kPa) à une température voisine de 20°C en présence de potasse en pastilles. On obtient ainsi 9,5 g de N-butyl (pyridyl-3)-3 1H,3H-pyrrolo [1,2-c] thiazolecarboxamide-7 sous forme de cristaux blancs fondant à 165°C.

L'acide (pyridyl-3)-3 1H,3H-pyrrolo [1,2-c] thiazolecarboxylique-7 est préparé comme à l'exemple 2.

*Exemple 10*

Une solution de 9,1 g d'acide (pyridyl-3)-3 1H,3H-pyrrolo [1,2-c] thiazolecarboxylique-7 et de 8,9 g de N,N'-carbonyldiimidazole dans 40 $cm^3$ de tétrahydrofuranne anhydre est agitée sous azote sec à une température voisine de 25°C pendant 1 heure et 45 minutes. A la solution obtenue, on ajoute en 20 minutes à une température comprise entre 25°C et 29°C une solution de 9,6 g de N,N-diéthylamino-3 propylamine dans 15 $cm^3$ de tétrahydrofuranne anhydre. La solution obtenue est agitée à une température voisine de 25°C pendant encore 2 heures et 20 minutes puis on ajoute 30 $cm^3$ d'eau distillée au mélange réactionnel. Le solvant est évaporé sous pression réduite (20 mm de mercure; 2,7 kPa) à une température voisine de 50°C et le résidu est repris par 500 $cm^3$ d'eau distillée. Un produit cristallise; la suspension est conservée à une température voisine de 4°C pendant 16 heures. Les cristaux sont séparés par filtration, lavés 10 fois par 800 $cm^3$ au total d'eau distillée et séchés sous pression réduite (20 mm de mercure; 2,7 kPa) à une température voisine de 20°C en présence de potasse en pastilles. On obtient ainsi 11 g de produit. Ce produit est réuni à 3 g de produit préparé de la même façon dans une autre opération antérieure et est dissous dans 80 $cm^3$ d'un mélange bouillant d'acétate d'éthyle et de cyclohexane (50-50 en volumes). La solution obtenue est additionnée de 0,5 g de noir décolorant et filtrée à chaud. Le filtrat est refroidi à une température voisine de 4°C pendant 1 heure et 30 minutes. Les cristaux apparus sont séparés par filtration, lavés 2 fois par 10 $cm^3$ au total d'un mélange d'acétate d'éthyle et de cyclohexane (50-50 en volumes) refroidi à une température voisine de 4°C, et séchés sous pression réduite (20 mm de mercure; 2,7 kPa) à une température voisine de 20°C en présence de potasse en pastilles. On obtient ainsi 10,9 g de N-(diéthylamino-3 propyl) (pyridyl-3)-3 1H,3H-pyrrolo [1,2-c] thiazolecarboxamide-7 sous forme de cristaux blancs fondant à 119°C.

L'acide (pyridyl-3)-3 1H,3H-pyrrolo [1,2-c] thiazolecarboxylique-7 est préparé comme à l'exemple 2.

*Exemple 11*

A une solution de 5 g de {[(pyridyl-3)-3 1H,3H-pyrrolo [1,2-c] thiazolyl-7] carbonylamino}-6 hexanoate d'éthyle dans 60 $cm^3$ d'éthanol, on ajoute en 5 minutes à une température comprise entre 20°C et 30°C, 13 $cm^3$ d'une solution aqueuse de soude 2N. La solution est agitée à une température voisine de 20°C pendant 16 heures puis est concentrée à sec sous pression réduite (20 mm de mercure; 2,7 kPa) à une température voisine de 40°C. Le résidu obtenu est dissous dans 50 $cm^3$ d'eau distillée et la solution obtenue est extraite 2 fois par 40 $cm^3$ au total d'acétate d'éthyle, additionnée de 0,5 g de noir décolorant, filtrée et amenée à un pH voisin de 4 par addition d'une solution aqueuse d'acide chlorhydrique 0,5N. Les cristaux apparus sont séparés par filtration, lavés 5 fois par 100 $cm^3$ au total d'eau distillée et séchés sous pression réduite (20 mm de mercure; 2,7 kPa) à une température voisine de 20°C en présence de potasse en pastilles. On obtient ainsi 4,1 g de produit brut fondant à 148°C. Ce produit est dissous dans 65 $cm^3$ d'éthanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 6 $cm^3$ au total d'éthanol refroidi à une température voisine de 4°C et 3 fois par 15 $cm^3$ au total d'éther diéthylique puis séchés sous pression réduite (20 mm de mercure; 2,7 kPa) à une température voisine de 20°C en présence de potasse en pastilles. On obtient ainsi 3,4 g d'acide {[(pyridyl-3)-3 1H,3H-pyrrolo [1,2-c] thiazolyl-7] carbonylamino}-6 hexanoïque sous forme de cristaux blancs fondant à 165°C.

Le { [(pyridyl-3)-3 1H,3H-pyrrolo [1,2-c] thiazolyl-7] carbonylamino}-6 hexanoate d'éthyle est préparé de la manière suivante:

A une suspension de 6,15 g de chlorhydrate de chloroformyl-7 (pyridyl-3)-3 1H,3H-pyrrolo [1,2-c] thiazole dans 50 $cm^3$ de dichloro-1,2 éthane, on ajoute en 25 minutes à une température comprise entre 21°C et 33°C une solution de 3,9 g d'amino-6 hexanoate d'éthyle et de 4,95 g de triéthylamine dans 100 $cm^3$ de dichloro-1,2 éthane. La solution obtenue est agitée à une température voisine de 20°C pendant 16 heures puis est diluée par 100 $cm^3$ de chlorure de méthylène, lavés 1 fois par 60 $cm^3$ d'une solution aqueuse de soude N et 5 fois par 500 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 7,8 g de produit brut. Ce produit est chromatographié sur une colonne de 6 cm de diamètre contenant 480 g de silice (0,04-0,063 mm). On élue par des mélanges d'acétate d'éthyle et de méthanol sous une pression de 0,5 bar (51 kPa) et en recueillant des fractions de 100 $cm^3$. Les 18 premières fractions provenant de l'élution par de l'acétate d'éthyle pur et les 5 fractions suivantes provenant de l'élution par un mélange d'acétate d'éthyle et de méthanol (90-10 en volumes) sont éliminées, les 15 fractions suivantes provenant de l'élution par un mélange d'acétate d'éthyle et de méthanol (90-10 en volumes) sont réunis et concentrées à sec sous pression réduite (20 mm de mercure; 2,7 kPa) à une température voisine de 50°C. On obtient ainsi 5 g de {[(pyridyl-3)-3 1H,3H-pyrrolo [1,2-c] thiazolyl-7] carbonylamino}-6 hexanoate d'éthyle sous forme de cristaux crème fondant à 126°C.

Le chlorhydrate de chloroformyl-7 (pyridyl-3)-3 1H,3H-pyrrolo [1,2-c] thiazole est préparé de la manière suivante:

Une suspension de 9,8 g d'acide (pyridyl-3)-3 1H,3H-pyrrolo [1,2-c] thiazolecarboxylique-7 dans un mélange de 23,8 g de chlorure de thionyle, de 0,1 $cm^3$ de diméthylformamide et de 100 $cm^3$ de dichloro-1,2 éthane est chauffée à l'ébullition pendant 1 heure et 15 minutes. Après refroidissement du mélange réactionnel, le solvant est évaporé sous pression réduite (20 mm de mercure; 2,7 kPa) à une température voisine de 50°C. Le résidu obtenu est mis en suspension dans 100 $cm^3$ de cyclohexane anhydre et les cristaux sont séparés par filtration, lavés 2 fois par 10 $cm^3$ au total de cyclohexane anhydre et séchés sous pression réduite (20 mm de mercure; 2,7 kPa) à une température voisine de 20°C. On obtient ainsi 12,1 g de chlorhydrate de chloroformyl-7 (pyridyl-3)-3 1H,3H-pyrrolo [1,2-c] thiazole sous forme de cristaux ocre fondant à 185°C.

L'acide (pyridyl-3)-3 1H,3H-pyrrolo [1,2-c] thiazolecarboxylique-7 est préparé comme à l'exemple 2.

L'amino-6 hexanoate d'éthyle peut être préparé d'après C.S. MARVEL, J.R. ELLIOTT, F.E. BOETTNER et H. YUSKA, J. Amer. Chem. Soc., 68, 1681 (1946).

*Exemple 12*

Une solution de 11,3 g d'acide (pyridyl-3)-3 1H,3H-pyrrolo [1,2-c] thiazolecarboxylique-7 et de 11,2 g de N,N'-carbonyldiimidazole dans 150 $cm^3$ de tétrahydrofuranne anhydre est agitée sous azote sec à une température voisine de 20°C pendant 1 heure et 20 minutes. A la solution obtenue, on ajoute en 15 minutes à une température comprise entre 25°C et 29°C une solution de 15,6 g d'(Amino-2 éthyl)-1 (pyridyl-2)-4 pipérazine dans 30 $cm^3$ de tétrahydrofuranne anhydre. On agite la solution obtenue à une température voisine de 25°C pendant 4 heures et 30 minutes puis ajoute 30 $cm^3$ d'eau distillée. Le solvant est évaporé sous pression réduite (20 mm de mercure; 2,7 kPa) à une température voisine de 50°C et le résidu est dissous dans 500 $cm^3$ d'acétate d'éthyle. La solution obtenue est lavée 6 fois par 600 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 50°C. Le résidu obtenu est dissous dans 50 $cm^3$ d'acétonitrile bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 4 fois par 40 $cm^3$ au total d'acétonitrile refroidi à une température voisine de 4°C, et séchés sous pression réduite (20 mm de mercure; 2,7 kPa) à une température voisine de 20°C en présence de potasse en pastilles. On obtient ainsi 17,8 g de produit fondant à 140°C. Ce produit est chromatographié sur une colonne de 6 cm de diamètre contenant 480 g de silice (0,040-0,063 mm) en éluant par un mélange d'acétate d'éthyle et de méthanol (80-20 en volumes) sous une pression de 0,5 bar (51 kPa) et en recueillant des fractions de 100 $cm^3$. Les 11 premières fractions sont éliminées, les 21 suivantes sont réunis et concentrées à sec sous pression réduite (20 mm de mercure; 2,7 kPa) à une température voisine de 50°C. On obtient ainsi 16 g de produit. Ce produit est dissous dans 40 $cm^3$ d'isopropanol bouillant. La solution obtenue est filtrée à chaud et le filtrat est refroidi à une température voisine de 4°C pendant 1 heure. Les cristaux apparus sont séparés par filtration, lavés 3 fois par 10 $cm^3$ au total d'isopropanol refroidi à une température voisine de 4°C et séchés sous pression réduite (20 mm de mercure; 2,7 kPa) à une température voisine de 20°C en présence de potasse en pastilles. On obtient ainsi 14,7 g de N-{[(pyridyl-2)-4 pipérazinyl-1]-2 éthyl} (pyridyl-3)-3 1H,3H-pyrrolo [1,2-c] thiazolecarboxamide-7 sous forme de cristaux blancs fondant à 141°C.

L'acide (pyridyl-3)-3 1H,3H-pyrrolo [1,2-c] thiazolecarboxylique-7 est préparé comme à l'exemple 2.

L'(amino-2 éthyl)-1 (pyridyl-2)-4 pipérazine peut être préparé selon R.P. MULL et coll., J. Med. Pharm. Chem., 5, 944 (1962).

*Exemple 13*

Une solution de 7,4 g d'acide (pyridyl-3)-3 1H,3H-pyrrolo [1,2-c] thiazolecarboxylique-7 et de 7,3 g de N,N'-carbonyldiimidazole dans 120 $cm^3$ de tétrahydrofuranne anhydre est agitée sous azote sec à une température voisine de 20°C pendant 1 heure et 15 minutes. A la solution obtenue on ajoute en 10 minutes, à une température voisine de 10°C, une solution de 7,2 g de benzylamine dans 20 $cm^3$ de tétrahydrofuranne anhydre. Après 10 minutes d'agitation à une température voisine de 20°C, on observe une précipitation. Après 16 heures d'agitation à une température voisine de 20°C, on ajoute 30 $cm^3$ d'eau distillée au mélange réactionnel et évapore le solvant sous pression réduite (20 mm de mercure; 2,7 kPa) à une température voisine de 40°C. Le résidu obtenu est mis en suspension dans 250 $cm^3$ d'eau distillée. Les cristaux apparus sont séparés par filtration, lavés 2 fois par 20 $cm^3$ au total d'éthanol et 3 fois par 45 $cm^3$ au total d'éther diéthylique puis séchés sous pression réduite (20 mm de mercure; 2,7 kPa) à une température voisine de 20°C en présence de potasse en pastilles. On obtient ainsi 10,5 g de produit brut. Ce produit est dissous dans 480 $cm^3$ d'éthanol bouillant. La solution obtenue est additionnée de 0,5 g de noir décolorant et filtrée à chaud. Le filtrat est

refroidi à une température voisine de 4 °C pendant 1 heure. Les cristaux apparus sont séparés par filtration, lavés 2 fois par 20 $cm^3$ au total d'éthanol refroidi à une température voisine de 4 °C et 3 fois par 30 $cm^3$ au total d'éther diéthylique puis séchés sous pression réduite (20 mm de mercure; 2,7 kPa) à une température voisine de 20 °C en présence de potasse en pastilles. On obtient ainsi 8,2 g de N-benzyl (pyridyl-3)-3 1H,3H-pyrrolo [1,2-c] thiazolecarboxmide-7 sous forme de cristaux blancs fondant à 200 °C.

L'acide (pyridyl-3)-3 1H,3H-pyrrolo [1,2-c] thiazolecarboxylique-7 est préparé comme à l'exemple 2.

*Exemple 14*

Une solution de 4,9 g d'acide (pyridyl-3)-3 1H,3H-pyrrolo [1,2-c] thiazolecarboxylique-7 et de 4,9 g de N,N'-carbonyldiimidazole dans 80 $cm^3$ de tétrahydrofuranne anhydre est agitée sous azote sec à une température voisine de 20 °C pendant 3 heures. A la solution obtenue on ajoute en 5 minutes à une température voisine de 15 °C une solution de 5 g d'histamine dans 60 $cm^3$ de dimethylformamide. La solution obtenue est agitée à une température voisine de 20 °C pendant 20 heures. On ajoute alors 30 $cm^3$ d'eau distillée au mélange réactionnel et évapore le solvant sous pression réduite (5 mm de mercure; 0,7 kPa) à une température voisine de 50 °C. Le résidu huileux obtenu est repris par 350 $cm^3$ d'eau distillée. Un produit cristalline. La suspension obtenue est agitée à une température voisine de 4 °C pendant 1 heure. Les cristaux apparus sont séparés par filtration, lavés 3 fois par 30 $cm^3$ au total d'eau distillée refroidie à une température voisine de 4 °C et séchés sous pression réduite (20 mm de mercure; 2,7 kPa) à une température voisine de 20 °C en présence de potasse en pastilles. On obtient ainsi 6,6 g de produit fondant à 198 °C. Ce produit est réuni à 5,3 g de produit préparé de la même façon dans une autre opération antérieure, et dissous dans 100 $cm^3$ d'un mélange bouillant d'éthanol et d'eau distillée (50-50 en volumes). La solution obtenue est additionnée de 0,5 g de noir décolorant et filtrée à chaud. Le filtrat est refroidi à une température voisine de 4 °C pendant 1 heure. Les cristaux apparus sont séparés par filtration, lavés 2 fois par 20 $cm^3$ au total d'un mélange d'éthanol et d'eau distillée (50-50 en volumes), 2 fois par 20 $cm^3$ au total d'éthanol et 2 fois par 40 $cm^3$ au total d'éther diéthylique puis séchés sous pression réduite (20 mm de mercure; 2,7 kPa) à une température voisine de 20 °C en présence de potasse en pastilles. On obtient ainsi 10,6 g de N-[(imidazolyl-4)-2 éthyl] (pyridyl-3)-3 1H,3H-pyrrolo [1,2-c] thiazolecarboxamide-7 sous forme de cristaux crème fondant à 210 °C.

L'acide (pyridyl-3)-3 1H,3H-pyrrolo [1,2-c] thiazolecarboxylique-7 est préparé comme à l'exemple 2.

*Exemple 15*

A une solution de 11,3 g d'aniline et de 16,2 g de triéthylamine dans 300 $cm^3$ de dichloro-1,2 éthane on ajoute en 1 heure à une température comprise entre 25 °C et 34 °C 12,1 g de chlorhydrate de chloroformyl-7 (pyridyl-3)-3 1H,3H-pyrrolo [1,2-c] thiazole. La suspension obtenue est agitée à une température voisine de 20 °C pendant 16 heures. Le mélange réactionnel est alors lavé 3 fois par 300 $cm^3$ au total d'une solution aqueuse de soude 2N et 7 fois par 1400 $cm^3$ au total d'eau distillée. La phase organique est extraite 3 fois par 300 $cm^3$ au total d'une solution aqueuse d'acide chlorhydrique 5N et les extraits aqueux sont réunis, lavés 3 fois par 150 $cm^3$ au total de chlorure de méthylène, additionnés de 0,5 g de noir décolorant et filtrés. Le filtrat est amené à un pH voisin de 10 par addition d'une solution aqueuse de soude 10N et extrait 3 fois par 450 $cm^3$ au total de chlorure de méthylène. Les extraits organiques sont réunis, lavés 3 fois par 600 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 50 °C. On obtient ainsi 16,2, g de produit huileux. Ce produit est dissous dans 60 $cm^3$ d'un mélange bouillant d'acétate d'éthyle et de cyclohexane (50-50 en volumes). La solution obtenue est refroidi à une température voisine de 4 °C pendant 30 minutes. Les cristaux apparus sont séparés par filtration, lavés 3 fois par 20 $cm^3$ au total d'un mélange d'acétate d'éthyle et de cyclohexane (50-50 en volumes) et séchés sous pression réduite (20 mm de mercure; 2,7 kPa) à une température voisine de 20 °C en présence de potasse en pastilles. On obtient 5,8 g de produit fondant à 133 °C. Ce produit est réuni à 0,6 g de produit préparé de la même manière dans une autre opération antérieure et est dissous dans 110 $cm^3$ d'un mélange bouillant d'acétate d'éthyle et de cyclohexane (50-50 en volumes). La solution obtenue est additionnée de 0,5 g de noir décolorant et filtrée à chaud. Le filtrat est refroidi à une température voisine de 4 °C pendant 1 heure. Les cristaux apparus sont séparés par filtration, lavés 2 fois par 10 $cm^3$ d'un mélange d'acétate d'éthyle et de cyclohexane (50-50 envolumes) refroidi à une température voisine de 4 °C et séchés sous pression réduite (20 mm de mercure; 2,7 kPa) à une température voisine de 20 °C en présence de potasse en pastilles. On obtient ainsi 5,3 g de N-phényl (pyridyl-3)-3 1H,3H-pyrrolo [1,2-c] thiazolecarboxamide-7 sous forme de cristaux blancs fondant à 135 °C.

Le chlorhydrate de chloroformyl-7 (pyridyl-3)-3 1H,3H-pyrrolo [1,2-c] thiazole est préparé comme à l'exemple 11.

*Exemple 16*

A une solution de 5,1 g de chloro-4 aniline et de 8,1 g de triéthylamine dans 150 $cm^3$ de dichloro-1,2 éthane on ajoute par petites portions à la fois en 15 minutes à une température voisine de 20 °C, 6 g de chlorhydrate de chloroformyl-7 (pyridyl-3)-3 1H,3H--pyrrolo [1,2-c] thiazole. La solution est agitée à une température voisine de 20 °C pendant 16 heures puis est diluée par 100 $cm^3$ de chlorure de méthylène, lavée 2 fois par 100 $cm^3$ au total d'eau distillée, 2 fois par 100 $cm^3$ au total d'une solution aqueuse de soude 2N et 5 fois par 500 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de

50°C. On obtient ainsi 9 g de solide brut. Ce produit est dissous dans 150 $cm^3$ d'acétonitrile bouillant et la solution obtenue est additionnée de 0,5 g de noir décolorant et filtrée à chaud. Le filtrat est refroidi à une température voisine de 4°C pendant 16 heures. Les cristaux apparus sont séparés par filtration, lavés 2 fois par 15 $cm^3$ au total d'acétonitrile refroidi à une température voisine de 4°C et séchés sous pression réduite (20 mm de mercure; 2,7 kPa) à une température voisine de 20°C en présence de potasse en pastilles. On obtient ainsi 4,3 g de N-(chloro-4 phényl (pyridyl-3)-3 1H,3H-pyrrolo [1,2-c] thiazolecarboxamide-7 sous forme de cristaux crème fondant à 187°C.

Le chlorhydrate de chloroformyl-7 (pyridyl-3)-3 1H,3H-pyrrolo [1,2-c] thiazone est préparé comme à l'exemple 11.

*Exemple 17*

Une solution de 2 g d'acide (pyridyl-3)-3 1H,3H-pyrrolo [1,2-c] thiazolecarboxylique-7 et de 1,45 g de N,N'-carbonyldiimidazole dans 40 $cm^3$ de tétrahydrofuranne anhydre est agitée à une température voisine de 20°C pendant 4 heures. La solution obtenue est additionnée de 0,5 g de noir décolorant, filtrée et concentrée à sec sous pression réduite (20 mm de mercure; 2,7 kPa) à une température voisine de 45°C. Le résidu est repris par 100 $cm^3$ d'eau distillée et la suspension obtenue est agitée à une température voisine de 20°C pendant 30 minutes. Les cristaux sont séparés par filtration, lavés 3 fois par 60 $cm^3$ au total d'eau distillée et séchés sous pression réduite (20 mm de mercure; 2,7 kPa) à une température voisine de 20°C en présence de potasse en pastilles. On obtient ainsi 2 g de produit brut. Ce produit est dissous dans 35 $cm^3$ d'isopropanol bouillant. La solution obtenue est refroidie à une température voisine de 4°C pendant 1 heure. Les cristaux apparus sont séparés par filtration, lavés 2 fois par 20 $cm^3$ au total d'isopropanol refroidi à une température voisine de 4°C et 2 fois par 20 $cm^3$ au total d'oxyde d'isopropyle puis séchés sous pression réduite (20 mm de mercure; 2,7 kPa) à une température voisine de 20°C en présence de potasse en pastilles. On obtient ainsi 1,7 g d'(imidazolyl-1 carbonyl)-7 (pyridyl-3)-3 1H,3H-pyrrolo [1,2-c] thiazole sous forme de cristaux crème fondant à 117°C.

L'acide (pyridyl-3)-3 1H,3H-pyrrolo [1,2-c] thiazolecarboxylique-7 est préparé comme à l'exemple 2.

*Exemple 18*

On chauffe pendant 5 heures et 30 minutes à une température voisine de 150°C une solution de 16 g d'(imidazolyl-1 carbonyl)-7 (pyridyl-3)-3 1H,3H-pyrrolo [1,2-c] thiazole et de 10,2 g d'amino-2 pyridine dans 150 $cm^3$ de diméthylformamide anhydre. La solution est alors concentrée à sec sous pression réduite (20 mm de mercure; 2,7 kPa) à une température voisine de 80°C et l'huile résiduelle est reprise par 500 $cm^3$ d'eau distillée. Des cristaux apparaissent. La suspension est agitée à une température voisine de 20°C pendant 16 heures. Les cristaux sont séparés par filtration, lavés 3 fois par 300 $cm^3$ au total d'eau distillée et séchés sous pression réduite (20 mm de mercure; 2,7 kPa) à une température voisine de 20°C en présence de potasse en pastilles. On obtient ainsi 14,6 g de produit brut fondant à 141°C. Ce produit est dissous dans 75 $cm^3$ d'éthanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 10 $cm^3$ au total d'éthanol refroidi à une température voisine de 4°C et 3 fois par 15 $cm^3$ au total d'éther diéthylique puis séchés sous pression réduite (20 mm de mercure; 2,7 kPa) à une température voisine de 20°C en présence de potasse en pastilles. On obtient ainsi 11,6 g de N-(pyridyl-2) (pyridyl-3)-3 1H,3H-pyrrolo [1,2-c] thiazolecarboxamide-7 sous forme de cristaux beiges fondant à 145°C.

L'(imidazolyl-1 carbonyl)-7 (pyridyl-3)-3 1H,3H-pyrrolo [1,2-c] thiazole est préparé comme à l'exemple 17.

*Exemple 19*

A une solution de 3,8 g d'amino-3 pyridine et de 8,1 g de triéthylamine dans 150 $cm^3$ de dichloro-1,2 éthane, on ajoute par petites portions à la fois, en 20 minutes, à une température comprise entre 19°C et 26°C, 6 g de chlorhydrate de chloroformyl-7 (pyridyl-3)-3 1H,3H-pyrrolo [1,2-c] thiazole. La suspension obtenue est agitée à une température voisine de 20°C pendant 16 heures. Les cristaux sont séparés par filtration, lavés 3 fois par 75 $cm^3$ au total de dichloro-1,2 éthane, 3 fois par 75 $cm^3$ au total d'oxyde d'isopropyle puis 5 fois par 150 $cm^3$ au total d'eau distillée et séchés à l'air. On obtient ainsi 6 g de produit brut fondant à 210°C. Ce produit est repris par 75 $cm^3$ d'éthanol bouillant. La solution trouble obtenue est additionnée de 0,5 g de noir décolorant et filtrée à chaud. Le filtrat est refroidi à une température voisine de 4°C pendant 16 heures. Les cristaux apparus sont séparés par filtration, lavés 3 fois par 15 $cm^3$ au total d'éthanol puis 3 fois par 45 $cm^3$ au total d'éther diéthylique et séchés sous pression réduite (20 mm de mercure; 2,7 kPa) à une température voisine de 20°C en présence de potasse en pastilles. On obtient ainsi 2,5 g de N-pyridyl-3 (pyridyl-3)-3 1H,3H-pyrrolo [1,2-c] thiazolecarboxamide-7 sous forme de cristaux blancs fondant à 220°C.

Le chlorhydrate de chloroformyl-7 (pyridyl-3)-3 1H,3H-pyrrolo [1,2-c] thiazole est préparé comme à l'exemple 11.

*Exemple 20*

Une solution de 10 g d'acide (pyridyl-3)-3 1H,3H-pyrrolo [1,2-c] thiazolecarboxylique-7 est de 9,9 g de N,N'-carbonyldiimidazole dans 160 $cm^3$ de tétrahydrofuranne anhydre est agité sous azote sec à une température voisine de 20°C pendant 1 heure et 15 minutes. A la solution obtenue on ajoute en 10 minutes, à une température voisine de 15°C, 6,1 g d'hydrate d'hydrazine. Après 1 heure d'agitation à une température voisine de 20°C, on ajoute 100 $cm^3$ d'eau distillée au mélange réactionnel et évapore le solvant sous pression réduite (20 mm de mercure; 2,7 kPa) à une température voisine de 55°C. Le solide obtenu est mis en suspension dans 400 $cm^3$

d'eau distillée. Après 15 minutes d'agitation à une température voisine de 4 °C, les cristaux apparus sont séparés par filtration, lavés 3 fois par 150 $cm^3$ au total d'eau distillée, 3 fois par 30 $cm^3$ au total d'éther refroidi à une température voisine de 4 °C et 3 fois par 45 $cm^3$ au total d'éther diéthylique, puis séchés sous pression réduite (20 mm de mercure; 2,7 kPa) à une température voisine de 20 °C en présence de potasse en pastilles. On obtient ainsi 9 g de produit brut fondant à 185 °C. Ce produit, réuni avec 3,9 g de produit préparé de la même manière dans une autre opération antérieure, est dissous dans 350 $cm^3$ d'éthanol bouillant. La solution obtenue est additionnée de 0,5 g de noir décolorant et filtrée à chaud. Le filtrat est refroidi à une température voisine de 4 °C pendant 1 heure. Les cristaux apparus sont séparés par filtration, lavés 3 fois par 30 $cm^3$ au total d'éthanol refroidi à une température voisine de 4 °C et 3 fois par 30 $cm^3$ au total d'éther diéthylique puis séchés sous pression réduite (20 mm de mercure; 2,7 kPa) à une température voisine de 20 °C en présence de potasse en pastilles. On obtient ainsi 11,2 g de produit fondant à 186 °C. Ce produit est dissous dans 300 $cm^3$ d'éthanol bouillant. La solution obtenue est refroidie à une température voisine de 4 °C pendant 30 minutes. Les cristaux apparus sont séparés par filtration, lavés 3 fois par 15 $cm^3$ au total d'éthanol refroidi à une température voisine de 4 °C et 3 fois par 15 $cm^3$ au total d'éther diéthylique puis séchés sous pression réduite (20 mm de mercure; 2,7 kPa) à une température voisine de 20 °C en présence de potasse en pastilles. On obtient ainsi 10,4 g de (pyridyl-3)-3 1H,3H-pyrrolo [1,2-c] thiazolecarbohydrazine-7 sous forme de cristaux crème fondant à 187 °C.

L'acide (pyridyl-3)-3 1H,3H-pyrrolo [1,2-c] thiazolecarboxylique-7 est préparé comme à l'exemple 2.

*Esemple 21*

Une solution de 5 g d'acide (pyridyl-3)-3 1H,3H-pyrrolo [1,2-c] thiazolecarboxylique-7 et de 4,95 g de N,N'-carbonyldiimidazole dans 50 $cm^3$ de tétrahydrofuranne anhydre est agité à une température voisine de 20 °C pendant 1 heure. On ajoute ensuite en 20 minutes à une température voisine de 25 °C une solution de 4,7 g d'(hydroxy-2 éthyl)-1 pipérazine dans 50 $cm^3$ de tétrahydrofuranne. La solution est agitée à une température voisine de 20 °C pendant 16 heures et est alors additionnée de 40 $cm^3$ d'eau distillée. Le solvant est évaporé sous pression réduite (20 mm de mercure; 2,7 kPa) à une température voisine de 45 °C. Le résidu obtenu est dissous dans 350 $cm^3$ de chlorure de méthylène. La solution obtenue est lavée 2 fois par 200 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 50 °C. On obtient ainsi 7,2 g de produit brut. Ce produit est chromatographié sur une colonne de 6 cm de diamètre contenant 480 g de silice (0,04-0,063 mm). On élue par un mélange (9-1 en volumes) d'acétonitrile et d'ammoniaque (d = 0,92) sous une pression de 0,5 bar (51 kPa) en recueillant des fractions de 100 $cm^3$. Les 15 premières fractions sont éliminées; la fraction suivante est concentrée à sec sous pression réduite (20 mm de mercure; 2,7 kPa) à une température voisine de 60 °C. On obtient 1,4 g de produit. Ce produit est dissous dans 20 $cm^3$ d'éthanol et la solution obtenue est ajoutée en 35 minutes à 20 $cm^3$ d'une solution éthanolique de gaz chlorhydrique 5N. La suspension obtenue est abandonnée à une température voisine de 20 °C pendant 3 jours. Les cristaux sont séparés par filtration, lavés 2 fois par 10 $cm^3$ au total d'éthanol et 2 fois par 20 $cm^3$ au total d'éther diéthylique puis séchés sous pression réduite (20 mm de mercure; 2,7 kPa) à une température voisine de 20 °C en présence de potasse en pastilles. On obtient ainsi 1,5 g d'[(hydroxy-2 éthyl)-4 pipérazinyl-1] carbonyl-7 (pyridyl-3)-3 1H,3H-pyrrolo [1,2-c] thiazole à l'état de trichlorhydrate, monohydrate sous forme de cristaux blancs fondant à 194 °C.

L'acide (pyridyl-3)-3 1H,3H-pyrrolo [1,2-c] thiazolecarboxylique-7 est préparé comme à l'exemple 2.

*Exemple 22*

On agite sous azote sec à une température voisine de 20 °C pendant 1 heure et 30 minutes une solution de 4,9 g d'acide (pyridyl-3)-3 1H,3H-pyrrolo [1,2-c] thiazolecarboxylique-7 et de 4,9 g de N,N'-carbonyldiimidazole dans 80 $cm^3$ de tétrahydrofuranne anhydre. A la solution obtenue on ajoute alors en 5 minutes à une température comprise entre 24 °C et 27 °C une solution de 5,3 g de benzyl-1 pipérazine dans 20 $cm^3$ de tétrahydrofuranne anhydre. La solution est agitée à une température voisine de 20 °C pendant 16 heures puis est filtrée et concentrée à sec sous pression réduite (20 mm de mercure; 2,7 kPa) à une température voisine de 40 °C. On obtient ainsi 13,9 g de produit brut que l'on chromatographie sur une colonne de 6 cm de diamètre contenant 480 g de silice (0,04-0,063 mm). On élue par un mélange de chlorure de méthylène et de méthanol (9-1 en volumes) sous une pression de 0,5 bar (51 kPa) en recueillant des fractions de 100 $cm^3$. Les 8 premières fractions sont éliminées, les 9 fractions suivantes sont réunis et concentrées à sec sous pression réduite (20 mm de mercure; 2,7 kPa) à une température voisine de 40 °C. On obtient ainsi 9 g de produit. Ce produit est dissous dans 90 $cm^3$ d'acétone. La solution obtenue est additionnée de 0,5 g de noir décolorant et filtrée. On ajoute alors à la solution une solution de 5,9 g d'acide oxalique dans 60 $cm^3$ d'acétone. La suspension obtenue est agitée à une température voisine de 20 °C pendant 30 minutes et les cristaux sont séparés par filtration, lavés 5 fois par 100 $cm^3$ au total d'acétone et séchés sous pression réduite (20 mm de mercure; 2,7 kPa) à une température voisine de 20 °C en présence de potasse en pastilles. On obtient ainsi 12,6 g de produit qui sont dissous dans 250 $cm^3$ d'eau distillée. La solution obtenue est amenée à un pH voisin de 12 par addition de 10 $cm^3$ d'une solution aqueuse de soude 10N et est extraite 3 fois par 300 $cm^3$ au total d'acétate d'éthyle. Les extraits organiques sont réunis, lavés 2 fois par 160 $cm^3$ au total d'eau distillée, séchés sur du sulfate de magnésium anhydre, additionnés de 0,5 g de noir décolorant, filtrés et concentrés à sec

sous pression réduite (20 mm de mercure; 2,7 kPa) à une température voisine de 40°C. On obtient ainsi 8 g de produit. Ce produit est dissous dans 50 $cm^3$ d'un mélange bouillant de cyclohexane et d'acétate d'éthyle (80-20 en volumes). La solution obtenue est additionnée de 0,5 g de noir décolorant et filtrée à chaud. Le filtrat est refroidi à une température voisine de 20°C pendant 3 heures. Les cristaux apparus sont séparés par filtration, lavés 2 fois par 20 $cm^3$ au total du mélange de cyclohexane et d'acétate d'éthyle (80-20 en volumes) et 1 fois par 15 $cm^3$ d'éther diéthylique puis séchés sous pression réduite (20 mm de mercure; 2,7 kPa) à une température voisine de 20°C en présence de potasse en pastilles. On obtient ainsi 3,6 g de (benzyl-4 pipérazinyl-1) carbonyl-7 (pyridyl-3)-3 1H,3H-pyrrolo [1,2-c] thiazole sous forme de cristaux blancs fondant à 95°C.

L'acide (pyridyl-3)-3 1H,3H-pyrrolo [1,2-c] thiazolecarboxylique-7 est préparé comme à l'exemple 2.

*Exemple 23*

Une solution de 7,4 g d'acide (pyridyl-3)-3 1H,3H-pyrrolo [1,2-c] thiazolecarboxylique-7 et de 7,3 g de N,N'-carbonyldiimidazole dans 120 $cm^3$ de tétrahydrofuranne anhydre est agitée sous azote sec à une température voisine de 20°C pendant 2 heures. A la solution on ajoute en 15 minutes, à une température voisine de 10°C, une solution de 15,3 g de N-phényl pipérazine dans 40 $cm^3$ de tétrahydrofuranne anhydre et maintient l'agitation pendant 3 heures à une température voisine de 20°C. On ajoute alors 30 $cm^3$ d'eau distillée au mélange réactionnel et évapore le solvant sous pression réduite (20 mm de mercure; 2,7 kPa) à une température voisine de 50°C. L'huile brute obtenue est dissous dans 100 $cm^3$ d'acétonitrile bouillant; la solution est additionnée de 0,5 g de noir décolorant et filtrée à chaud. Le filtrat est refroidi à une température voisine de 4°C pendant 1 heure. Les cristaux apparus sont séparés par filtration, lavés 3 fois par 25 $cm^3$ au total d'acétonitrile refroidi à une température voisine de 4°C et 3 fois par 45 $cm^3$ au total d'éther diéthylique puis séchés sous pression réduite (20 mm de mercure; 2,7 kPa) à une température voisine de 20°C en présence de potasse en pastilles. On obtient ainsi 8,1 g de produit fondant à 144°C. Ce produit, réuni avec 5,5 g de produit préparé de la même manière, dans une autre opération antérieure, est dissous dans 100 $cm^3$ d'éthanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 20 $cm^3$ au total d'éthanol refroidi à une température voisine de 4°C et 2 fois par 30 $cm^3$ au total d'éther diéthylique, et séchés sous pression réduite (20 mm de mercure; 2,7 kPa) à une température voisine de 20°C en présence de potasse en pastilles. On obtient ainsi 11,8 g de [(phényl-4 pipérazinyl-1) carbonyl]-7 (pyridyl-3)-3 1H,3H-pyrrolo [1,2-c] thiazole sous forme de cristaux blancs fondant à 146°C.

L'acide (pyridyl-3)-3 1H,3H-pyrrolo [1,2-c] thiazolecarboxylique-7 est préparé comme à l'exemple 2.

*Exemple 24*

On agite sous courant d'azote sec pendant 1 heure et 30 minutes une solution de 4,93 g d'acide (pyridyl-3)-3 1H,3H-pyrrolo [1,2-c] thiazolecarboxylique-7 et de 4,9 g de N,N'-carbonylimidazole dans 80 $cm^3$ de tétrahydrofuranne anhydre. A la solution obtenue on ajoute alors en 5 minutes à une température voisine de 25°C une solution de 4,9 g de (pyridyl-2)-1 pipérazine dans 20 $cm^3$ de tétrahydrofuranne anhydre. La solution obtenue est agitée à une température voisine de 20°C pendant 16 heures puis est diluée à une température voisine de 10°C par 90 $cm^3$ d'eau distillée et filtrée. Le filtrat est additionné à une température voisine de 4°C de 180 $cm^3$ d'eau distillée. La suspension obtenue est additionnée de 90 $cm^3$ d'eau distillée puis est agitée à une température voisine de 4°C pendant 1 heure. Les cristaux sont séparés par filtration, lavés 3 fois par 60 $cm^3$ au total d'eau distillée et séchés sous pression réduite (20 mm de mercure; 2,7 kPa) à une température voisine de 20°C en présence de potasse en pastilles. On obtient ainsi 7,05 g de produit brut fondant à 140°C. Ce produit est dissous dans 28 $cm^3$ d'éthanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 10 $cm^3$ au total d'éthanol refroidi à une température voisine de 4°C et 1 fois par 10 $cm^3$ d'éther diéthylique puis séchés sous pression réduite (20 mm de mercure; 2,7 kPa) à une température voisine de 20°C en présence de potasse en pastilles. On obtient ainsi 5,7 g de [(pyridyl-2)-4 pipérazinyl-1] carbonyl-7 (pyridyl-3)-3 1H,3H-pyrrolo [1,2-c] thiazole sous forme de cristaux crème fondant à 142°C.

L'acide (pyridyl-3)-3 1H,3H-pyrrolo [1,2-c] thiazolecarboxylique-7 est préparé comme à l'exemple 2.

*Exemple 25*

On agite sous courant d'azote sec pendant 1 heure à une température voisine de 20°C une solution de 5 g d'acide (pyridyl-3)-3 1H,3H-pyrrolo [1,2-c] thiazolecarboxylique-7 et de 5 g de N,N'-carbonyldiimidazole dans 50 $cm^3$ de tétrahydrofuranne anhydre. A la solution obtenue on ajoute en 15 minutes à une température voisine de 25°C une solution de 5,7 g de (pyrimidinyl-2)-1 pipérazine dans 50 $cm^3$ de tétrahydrofuranne anhydre. La solution est alors agitée à une température voisine de 20°C pendant 3 heures. On ajoute alors à la suspension obtenue 100 $cm^3$ d'eau distillée. Les cristaux sont séparés par filtration, lavés 5 fois par 250 $cm^3$ au total d'eau distillée, 5 fois par 25 $cm^3$ au total d'éthanol et 5 fois par 50 $cm^3$ au total d'éther diéthylique et dissous dans 250 $cm^3$ d'éthanol bouillant. La solution obtenue est additionnée de 0,5 g de noir décolorant et filtrée à chaud. Le filtrat est refroidi à une température voisine de 4°C pendant 72 heures. Les cristaux apparus sont séparés par filtration, lavés 2 fois par 30 $cm^3$ au total d'éthanol refroidi à une température voisine de 4°C et séchés sous pression réduite (20 mm de mercure; 2,7 kPa) à une température voisine de 20°C en présence de potasse en pastilles. On obtient ainsi 5,4 g de [(pyrimidinyl-2)-4 pipérazine-1] carbonyl-7 (pyridyl-3)-3 1H,3H-pyrrolo [1,2-c] thiazole sous forme de cristaux blancs fondant à 209°C.

L'acide (pyridyl-3)-3 1H,3H-pyrrolo [1,2-c] thiazolecarboxylique-7 est préparé comme à l'exemple 2.

*Exemple 26*

A une solution d'éthoxymagnésien du malonate de diéthyle dans 65 $cm^3$ d'un mélange d'éther et d'éthanol (3-1 en volumes), préparée à partir de 1,34 g de magnésium et de 8,8 g de malonate de diéthyle, on ajoute 5,1 g de triéthylamine; à la suspension obtenue, on ajoute en 15 minutes à une température comprise entre 25°C et 30°C 15 g de chlorhydrate de chloroformyl-7 (pyridyl-3)-3 1H,3H-pyrrolo [1,2-c] thiazole, puis on dilue par 35 $cm^3$ de tétrahydrofuranne anhydre et agite à une température voisine de 20°C pendant 16 heures. Le mélange réactionnel est alors repris par 25 $cm^3$ d'une solution aqueuse d'acide chlorhydrique 2N et extrait 5 fois par 750 $cm^3$ au total d'acétate d'éthyle. Les extraits organiques sont réunis, lavés 5 fois par 250 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 50°C. On obtient ainsi 19 g de produit fondant à 110°C. Ce produit est dissous dans un mélange de 25 $cm^3$ d'acide acétique, de 15 $cm^3$ d'eau distillée et de 3 $cm^3$ d'acide sulfurique concentré. La solution obtenue est chauffée à une température voisine de 100°C pendant 9 heures et 30 minutes puis est refroidie à une température voisine de 20°C, diluée par 150 $cm^3$ d'eau distillée, additionnée de 0,5 g de noir décolorant, filtrée, amenée à un pH voisin de 9 par addition de carbonate de sodium et extraite 3 fois par 450 $cm^3$ au total d'acétate d'éthyle. Les extraits organiques sont réunis, lavés 3 fois par 450 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 45°C. On obtient ainsi 9,5 g de produit. Ce produit est chromatographié sur une colonne de 6 cm de diamètre contenant 480 g de silice (0,04-0,063 mm) en éluant par des mélanges de cyclohexane et d'acétate d'éthyle sous une pression de 0,5 bar (51 kPa) et en recueillant des fractions de 200 $cm^3$. Les 9 premières fractions provenant de l'élution par un mélange d'acétate d'éthyle et de cyclohexane (80-20 en volumes) sont éliminées. Les 9 fractions suivantes provenant de l'élution par un mélange d'acétate d'éthyle et de cyclohexane (85-15 en volumes) sont réunis et concentrées à sec sous pression réduite (20 mm de mercure; 2,7 kPa) à une température voisine de 50°C. On obtient ainsi 7,2 g de produit. Ce produit est dissous dans 30 $cm^3$ d'isopropanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 3 fois par 15 $cm^3$ au total d'isopropanol refroidi à une température voisine de 4°C et séchés sous pression réduite (20 mm de mercure; 2,7 kPa) à une température voisine de 20°C en présence de potasse en pastilles. On obtient ainsi 5,7 g d'acétyl-7 (pyridyl-3)-3 1H,3H-pyrrolo [1,2-c] thiazole sous forme de cristaux crème fondant à 100°C.

Le chlorhydrate de chloroformyl-7 (pyridyl-3)-3 1H,3H-pyrrolo [1,2-c] thiazole est préparé comme à l'exemple 11.

L'éthoxymagnésien du malonate de diéthyle est préparé selon G.A. REYNOLDS et C.R. HAUSER, Org. Synth. Coll. Vol. 4, 708 (1963).

*Exemple 27*

Une solution de 2,6 g de (pyridyl-3)-3 1H,3H-pyrrolo [1,2-c] thiazolecarbothioamide-7 et de 6,95 g de chlorhydrate d'hydroxylamine dans 25 $cm^3$ de pyridine est additionnée de 2,7 g de chlorure mercurique et la suspension obtenue est agitée à une température voisine de 20°C pendant 48 heures puis est versée sur 280 $cm^3$ d'eau distillée. La suspension obtenue est chauffée à une température voisine de 80°C et filtrée. Le filtrat est concentré à sec sous pression réduite (20 mm de mercure; 2,7 kPa) à une température voisine de 70°C. Le résidu est dissous dans 100 $cm^3$ d'eau distillée et la solution obtenue est amenée à un pH voisin de 10 par addition d'une solution aqueuse de soude 10N puis extraite 3 fois par 300 $cm^3$ au total de chlorure de méthylène. Les extraits organiques sont réunis, lavés 2 fois par 100 $cm^3$ au total d'eau distillée, séchés sur du sulfate de magnésium anhydre, filtrés et concentrés à sec sous pression réduite (20 mm de mercure; 2,7 kPa) à une température voisine de 50°C. On obtient ainsi 2,5 g de produit brut. Ce produit est chromatographié sur une colonne de 3 cm de diamètre contenant 240 g de silice (0,04-0,063 mm). On élue par de l'acétate d'éthyle pur sous une pression de 0,5 bar (51 kPa) en recueillant des fractions de 50 $cm^3$. Les 12 premières fractions sont éliminées, les 11 fractions suivantes sont réunis et concentrées à sec sous pression réduite (20 mm de mercure; 2,7 kPa) à une température voisine de 50°C. On obtient ainsi 1,6 g de produit. Ce produit est dissous dans 35 $cm^3$ d'éthanol et la solution obtenue est additionnée de 2,6 $cm^3$ d'une solution éthanolique de gaz chlorhydrique 5,35N et est refroidie à une température voisine de 4°C pendant 16 heures. Les cristaux apparus sont séparés par filtration, lavés 2 fois par 15 $cm^3$ au total d'éthanol refroidi à une température voisine de 4°C et 3 fois par 45 $cm^3$ au total d'éther diéthylique puis séchés sous pression réduite (20 mm de mercure; 2,7 kPa) à une température voisine de 20°C en présence de potasse en pastilles. On obtient ainsi 1,6 g de dichlorhydrate de (pyridyl-3)-3 1H,3H-pyrrolo [1,2-c] thiazolecarboxamide-oxime-7 sous forme de cristaux blancs fondant à 206°C.

Le (pyridyl-3)-3 1H,3H-pyrrolo [1,2-c] thiazolecarbothioamide-7 peut être préparé de la façon suivante:

8,1 g de (pyridyl-3)-3 1H,3H-pyrrolo [1,2-c] thiazolecarboxamide-7 sont ajoutés à 100 $cm^3$ de pyridine saturée à une température voisine de 20°C par un courant gazeux de sulfure d'hydrogène. La suspension obtenue est additionnée de 7,3 g de pentasulfure de phosphore puis chauffée à l'ébullition pendant 2 heures sous courant gazeux de sulfure d'hydrogène. La solution obtenue est refroidie à une température voisine de 20°C puis est versée sur 1200 $cm^3$ d'eau distillée. La suspension obtenue est conservée à une température voisine de 4°C pendant 16 heures. Les cristaux sont séparés par filtration, lavés 5 fois par 500 $cm^3$ au total d'eau distillée, 5 fois par 25 $cm^3$ au total d'éthanol et 5 fois par 100 $cm^3$ au total d'éther diéthylique puis séchés sous pression réduite (20 mm de mercure; 2,7 kPa) à une température voisine de 20°C en présence de potasse en pastilles. On obtient ainsi 7,7 g de produit brut fondant à 205°C. Ce produit est dissous dans 180 $cm^3$ de butanol-1 bouillant. La solution obtenue est refroidie à une température voisine de 4°C pendant 1 heure. Les cristaux apparus sont séparés par filtration, lavés 3 fois par 15 $cm^3$ au total de butanol-1 refroidi à une température voisine de 4°C et 3 fois par 60 $cm^3$ au total d'éther diéthylique puis séchés sous pression réduite (20 mm de mercure; 2,7 kPa) à une température voisine de 20°C en présence de potasse en pastilles. On obtient ainsi 7,1 g de (pyridyl-3)-3 1H,3H-pyrrolo [1,2-c] thiazolecarbothioamide-7 sous forme de cristaux crème fondant à 205°C.

Le (pyridyl-3)-3 1H,3H-pyrrolo [1,2-c] thiazolecarboxamide-7 est préparé comme à l'exemple 3.

*Exemple 28*

Une suspension de 9,5 g d'iodhydrate de (pyridyl-3)-3 1H,3H-pyrrolo [1,2-c] thiazolethiocarboximidate-7 de S-méthyle et de 1,6 g de N,N-diméthylhydrazine dans 125 $cm^3$ d'éthanol est chauffée à une température voisine de 78°C pendant 4 heures et 30 minutes. La solution obtenue est concentrée à sec sous pression réduite (20 mm de mercure; 2,7 kPa) à une température voisine de 60°C. Le résidu obtenu est dissous dans 250 $cm^3$ d'eau distillée et la solution résultante est extraite 3 fois par 300 $cm^3$ au total d'acétate d'éthyle, additionnée de 0,5 g de noir décolorant, filtrée, amenée à un pH voisin de 10 par addition d'une solution aqueuse de soude 10N et extraite 3 fois par 300 $cm^3$ au total d'acétate d'éthyle. Les extraits organiques sont réunis, lavés 3 fois par 300 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 50°C. On obtient ainsi 4,8 g de produit brut que l'on chromatographie sur une colonne de 4 cm de diamètre contenant 320 g de silice (0,04-0,063 mm). On élue sous une pression de 0,5 bar (51 kPa) par des mélanges de chlorure de méthylène et de méthanol en recueillant des fractions de 100 $cm^3$. Les 13 premières fractions provenant de l'élution par un mélange de chlorure de méthylène et de méthanol (90-10 en volumes) sont éliminées. Les 4 fractions suivantes provenant de l'élution par un mélange de chlorure de méthylène et de méthanol (90-10 en volumes), les 9 fractions suivantes provenant de l'élution par un mélange de chlorure de méthylene et de méthanol (80-20 en volumes) et les 10 fractions suivantes provenant de l'élution par un mélange de chlorure de méthylène et de méthanol (50-50 en volumes) sont réunies et concentrées à sec sous pression réduite (20 mm de mercure; 2,7 kPa) à une température voisine de 60°C. On obtient ainsi 3,1 g de produit que l'on dissout dans 20 $cm^3$ d'éthanol. La solution trouble obtenue est filtrée et additionnée de 8,1 $cm^3$ d'une solution éthanolique de gaz chlorhydrique 5,35N; le chlorhydrate obtenu est précipité de sa solution par addition de 2 $cm^3$ d'éther diéthylique. Après refroidissement à une température voisine de 4°C pendant 16 heures, les cristaux sont séparés par filtration, lavés 5 fois par 75 $cm^3$ au total d'un mélange d'éther diéthylique et d'éthanol (50-50 en volumes) et 5 fois par 75 $cm^3$ au total d'éther diéthylique puis séchés sous pression réduite (20 mm de mercure; 2,7 kPa) à une température voisine de 20°C en présence de potasse en pastilles. On obtient ainsi 2,7 g de dichlorhydrate de (pyridyl-3)-3 1H,3H-pyrrolo [1,2-c] thiazolecarboxamide-7-diméthylhydrazone sous forme de cristaux crème fondant à 195°C.

L'iodhydrate de (pyridyl-3)-3 1H,3H-pyrrolo [1,2-c] thiazolecarboximidate-7 de S-méthyle peut être préparé de la façon suivante:

Une suspension de 7 g de (pyridyl-3)-3 1H,3H-pyrrolo [1,2-c] thiazolecarbothioamide-7 et de 4,2 g d'iodure de méthyle dans 250 $cm^3$ d'acétone est agitée à une température voisine de 20°C pendant 16 heures. On ajoute alors à la suspension 50 $cm^3$ de diméthylformamide et on poursuit l'agitation pendant 3 jours supplémentaires. Les cristaux sont alors séparés par filtration, lavés 3 fois par 90 $cm^3$ au total d'acétone et séchés sous pression réduite (20 mm de mercure; 2,7 kPa) à une température voisine de 20°C en présence de potasse en pastilles. On obtient ainsi 9,5 g d'iodhydrate de (pyridyl-3)-3 1H,3H-pyrrolo [1,2-c] thiazolethiocarboximidate-7 de S-méthyle sous forme de cristaux jaunes fondant à 193 à 194°C.

Le (pyridyl-3)-3 1H,3H-pyrrolo [1,2-c] thiazolecarbothioamide-7 est préparé comme à l'exemple 27.

*Exemple 29*

A une solution de 3,1 g de méthoxy-3 aniline dans 45 $cm^3$ de dioxanne chauffée à l'ébullition, on ajoute en 15 minutes un mélange de 3,8 g de chlorhydrate de chloroformyl-7 (pyridyl-3)-3 1H,3H-pyrrolo [1,2-c] thiazole et de 2,55 g de triéthylamine dans 100 $cm^3$ de dioxanne. La suspension obtenue est chauffée sous agitation à une température voisine de 100°C pendant 2 heures puis le solvant est évaporé sous pression réduite (20 mm de mercure; 2,7 kPa) à une température voisine de 50°C. Le résidu est repris par 150 $cm^3$ d'eau distillée et extrait 3 fois par 300 $cm^3$ au total de chlorure de méthylène. Les extraits organiques sont réunis, lavés 3 fois par 300 $cm^3$ au total d'eau distillée, 4 fois par 200 $cm^3$ au total d'une solution aqueuse de soude 2N et 4 fois par 400 $cm^3$ au total d'eau distillée puis séchés sur du sulfate de magnésium anhydre, additionnés de 0,5 g de noir décolorant, filtrés et concentrés à sec sous pression réduite (20 mm de mercure; 2,7 kPa) à une température voisine de 50°C. On obtient ainsi 5,5 g de produit que l'on chromatographie sur une colonne de 6 cm de diamètre contenant 480 g de silice (0,04-0,063 mm). On élue par des mélanges d'acétate d'éthyle et de cyclohexane sous une pression de 0,5 bar (51 kPa) en recueillant des fractions de 200 $cm^3$. Les 10 premières fractions provenant de l'élution par un mélange de cyclohexane et d'acétate d'éthyle (50-50 en volumes) et les 4 fractions suivantes provenant de l'élution par de l'acétate d'éthyle pur sont éliminées; les

12 fractions suivantes provenant de l'élution par de l'acétate d'éthyle pur sont réunis et concentrées à sec sous pression réduite (20 mm de mercure; 2,7 kPa) à une température voisine de 50°C. On obtient ainsi 3 g de produit brut. Ce produit est dissous dans 30 $cm^3$ d'isopropanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 10 $cm^3$ au total d'isopropanol refroidi à une température voisine de 4°C et 2 fois par 20 $cm^3$ au total d'oxyde d'isopropyle puis séchés sous pression réduite (20 mm de mercure; 2,7 kPa) à une température voisine de 20°C en présence de potasse en pastilles. On obtient ainsi 1,8 g de N-(méthoxy-3 phényl) (pyridyl--3)-3 1H,3H-pyrrolo [1,2-c] thiazolecarboxamide-7 sous forme de cristaux blancs fondant à 85°C.

Le chlorhydrate de chloroformyl-7 (pyridyl-3)-3 1H,3H-pyrrolo [1,2-c] thiazole est préparé comme à l'exemple 11.

*Exemple 30*

A une solution de 3,1 g de méthoxy-4 aniline et de 5,1 g de triéthylamine dans 50 $cm^3$ de dichloro-1,2 éthane, on ajoute en 15 minutes à une température comprise entre 21°C et 26°C, par petites portions à la fois, 3,8 g de chlorhydrate de chloroformyl-7 (pyridyl-3)-3 1H,3H-pyrrolo [1,2-c] thiazole. La suspension obtenue est agitée à une température voisine de 20°C pendant 16 heures. On ajoute alors au mélange réactionnel 300 $cm^3$ de chlorure de méthylène et 100 $cm^3$ d'eau distillée. La phase aqueuse est éliminée et la phase organique est lavée 2 fois par 200 $cm^3$ au total d'eau distillée, 3 fois par 240 $cm^3$ au total d'une solution aqueuse de soude N et 3 fois par 300 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 5,3 g de produit brut. Ce produit est dissous dans 35 $cm^3$ d'isopropanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 3 fois par 15 $cm^3$ au total d'isopropanol refroidi à une température voisine de 4°C et 3 fois par 30 $cm^3$ au total d'oxyde d'isopropyle puis séchés sous pression réduite (20 mm de mercure; 2,7 kPa) à une température voisine de 20°C en présence de potasse en pastilles. On obtient ainsi 1,7 g de N-(méthoxy-4 phényl) (pyridyl-3)-3 1H,3H-pyrrolo [1,2-c] thiazolecarboxamide-7 sous forme de cristaux crème fondant à 120°C.

Le chlorhydrate de chloroformyl-7 (pyridyl-3)-3 1H,3H-pyrrolo [1,2-c] thiazole est préparé comme à l'exemple 11.

*Exemple 31*

A une solution de 3,2 g de chloro-3 aniline et de 5,1 g de triéthylamine dans 50 $cm^3$ de dichloro-1,2 éthane, on ajoute en 15 minutes, à une température comprise entre 22° et 28°C, par petites portions à la fois, 3,8 g de chlorhydrate de chloroformyl-7 (pyridyl-3)-3 1H,3H-pyrrolo [1,2-c] thiazole. La suspension obtenue est agitée à une température voisine de 20°C pendant 16 heures. On ajoute alors au mélange réactionnel 300 $cm^3$ de dichloro-1,2 éthane et 100 $cm^3$ d'eau distillée. La phase aqueuse est éliminée et la phase organique est lavée 3 fois par 300 $cm^3$ au total d'eau distillée, 3 fois par 240 $cm^3$ au total d'une solution aqueuse de soude N puis 3 fois par 300 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 5,5 g de produit que l'on chromatographie sur une colonne de 6 cm de diamètre contenant 480 g de silice (0,04-0,063 mm). On élue par de l'acétate d'éthyle pur sous une pression de 0,5 bar (51 kPa) en recueillant des fractions de 100 $cm^3$. Les 11 premières fractions sont éliminées, les 8 fractions suivantes sont réunis et concentrées à sec sous pression réduite (20 mm de mercure; 2,7 kPa) à une température voisine de 50°C. On obtient ainsi 3,5 g de produit que l'on dissout dans 25 $cm^3$ d'isopropanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 10 $cm^3$ au total d'isopropanol refroidi à une température voisine de 4°C et 3 fois par 15 $cm^3$ au total d'oxyde d'isopropanol puis séchés sous pression réduite (20 mm de mercure; 2,7 kPa) à une température voisine de 20°C en présence de potasse en pastilles. On obtient ainsi 2,2 g de N-(chloro-3 phényl) (pyridyl-3)-3 1H,3H-pyrrolo [1,2-c] thiazolecarboxamide-7 sous forme de cristaux crème fondant à 150°C.

Le chlorhydrate de chloroformyl-7 (pyridyl-3)-3 1H,3H-pyrrolo [1,2-c] thiazole est préparé comme à l'exemple 11.

*Exemple 32*

A une solution de 2,7 g d'o-toluidine et de 5,1 g de triéthylamine dans 50 $cm^3$ de dichloro-1,2 éthane, on ajoute en 15 minutes à une température comprise entre 21°C et 27°C par petites portions à la fois, 3,8 g de chlorhydrate de chloroformyl-7 (pyridyl-3)-3 1H,3H-pyrrolo [1,2-c] thiazole. La suspension obtenue est agitée à une température voisine de 20°C pendant 3 jours. On ajoute alors au mélange réactionnel 300 $cm^3$ de dichloro-1,2 éthane et 100 $cm^3$ d'eau distillée. La phase aqueuse est éliminée et la phase organique est lavée 2 fois par 200 $cm^3$ au total d'eau distillée, 3 fois par 240 $cm^3$ au total d'une solution aqueuse de soude N et 3 fois par 300 $cm^3$ au total d'eau distillée, séchés sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 4,6 g de produit que l'on chromatographie sur une colonne de 6 cm de diamètre contenant 480 g de silice (0,04-0,063 mm). On élue par de l'acétate d'éthyle pur sous une pression de 0,5 bar (51 kPa) en recueillant des fractions de 100 $cm^3$. Les premières fractions sont éliminées, les 12 fractions suivantes sont réunies et concentrées à sec sous pression réduite (20 mm de mercure; 2,7 kPa) à une température voisine de 50°C. On obtient ainsi 2,9 g de produit brut fondant à 85°C. Ce produit est dissous dans 25 $cm^3$ d'isopropanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 10 $cm^3$ au total d'isopropanol, refroidi à une température voisine de 4°C, et 3 fois par 15 $cm^3$ au total d'oxyde d'isopropyle puis séchés sous pression réduite (20 mm de mercure; 2,7 kPa) à une température voisine de 20°C en présence de potasse en pastilles. On obtient ainsi 1,8 g de N-(o-tolyl) (pyridyl-3)-3 1H,3H-pyrrolo [1,2-c] thiazolecarboxamide-7 sous forme de cristaux crème fondant à 101°C.

Le chlorhydrate de chloroformyl-7 (pyridyl-3)-3 1H,3H-pyrrolo [1,2-c] thiazole est préparé comme à l'exemple 11.

*Exemple 33*

A une suspension de 6,7 g de dichlorhydrate de N,N-diméthyl p-phénylènediamine et de 13,1 g de triéthylamine dans 250 $cm^3$ de dichloro-1,2 éthane, on ajoute en 15 minutes, à une température comprise entre 21°C et 28°C, par petites portions à la fois, 4,8 g de chlorhydrate de chloroformyl-7 1H,3H-pyrrolo [1,2-c] thiazole. La suspension obtenue est agitée à une température voisine de 20°C pendant 16 heures. On ajoute au mélange réactionnel 300 $cm^3$ de dichloro-1,2 éthane et 100 $cm^3$ d'eau distillée. La phase aqueuse est éliminée et la phase organique est lavée 2 fois par 200 $cm^3$ au total d'eau distillée, 2 fois par 200 $cm^3$ au total d'une solution aqueuse de soude N et 3 fois par 300 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 6,8 g de produit brut que l'on dissout dans 90 $cm^3$ d'acétonitrile bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 3 fois par 30 $cm^3$ au total d'acétonitrile refroidi à une température voisine de 4°C et séchés sous pression réduite (20 mm de mercure; 2,7 kPa) à une température voisine de 20°C en présence de potasse en pastilles. On obtient ainsi 3 g de N-(N',N'-diméthylamino-4 phényl) (pyridyl-3)-3 1H,3H-pyrrolo [1,2-c] thiazolecarboxamide-7 sous forme de cristaux beiges fondant à 196°C.

Le chlorhydrate de chloroformyl-7 (pyridyl-3)-3 1H,3H-pyrrolo [1,2-c] thiazole est préparé comme à l'exemple 11.

*Exemple 34*

On agite sous azote sec pendant 2 heures et 30 minutes à une température voisine de 20°C une solution de 2 g d'acide (pyridyl-3)-3 1H,3H-pyrrolo [1,2-c] thiazolecarboxylique-7 et de 2,1 g de N,N'-carbonyldiimidazole dans 50 $cm^3$ de tétrahydrofuranne anhydre. A la solution obtenue, on ajoute en 10 minutes, à une température comprise entre 20°C et 25°C une solution de 2,9 g de (méthoxy-4 phényl)-1 pipérazine dans 50 $cm^3$ de tétrahydrofuranne anhydre. La solution obtenue est agitée à une température voisine de 20°C pendant 16 heures. On ajoute alors au mélange réactionnel 30 $cm^3$ d'eau distillée et on évapore le solvant sous pression réduite (20 mm de mercure; 2,7 kPa) à une température voisine de 50°C. Le résidu huileux obtenu est repris par 80 $cm^3$ d'eau distillée et extrait 3 fois par 200 $cm^3$ au total d'acétate d'éthyle. Les extraits organiques sont réunis, lavés 3 fois par 240 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 50°C. On obtient ainsi 4,5 g de produit brut que l'on dissout dans 45 $cm^3$ d'un mélange bouillant de cyclohexane et d'acétate d'éthyle (50-50 en volumes). La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 10 $cm^3$ au total d'un mélange de cyclohexane et d'acétate d'éthyle (50-50 en volumes) et séchés sous pression réduite (20 mm de mercure; 2,7 kPa) à une température voisine de 20°C en présence de potasse en pastilles. On obtient ainsi 2,1 g de [(méthoxy-4 phényl)-4 pipérazinyl-1 carbonyl]-7 (pyridyl-3)-3 1H,3H-pyrrolo [1,2-c] thiazole sous forme de cristaux crème fondant à 131°C.

L'acide (pyridyl-3)-3 1H,3H-pyrrolo [1,2-c] thiazolecarboxylique-7 peut être préparé comme à l'exemple 2.

*Exemple 35*

On agite sous azote sec pendant 2 heures à une température voisine de 25°C une solution de 4,9 g d'acide (pyridyl-3)-3 1H,3H-pyrrolo [1,2-c] thiazolecarboxylique-7 et de 4,9 g de N,N'-carbonyldiimidazole dans 80 $cm^3$ de tétrahydrofuranne anhydre, puis on ajoute en 5 minutes à une température comprise entre 25°C et 29°C une solution de 3 g de méthyl-4 pipérazine dans 20 $cm^3$ de tétrahydrofuranne anhydre. La solution obtenue est agitée à une température voisine de 20°C pendant 16 heures puis est filtrée et concentrée à sec sous pression réduite (20 mm de mercure; 2,7 kPa) à une température voisine de 45°C. Le résidu huileux brut obtenu est chromatographié sur une colonne de 6 cm de diamètre contenant 480 g de silice (0,04-0,063 mm). On élue par un mélange de chlorure de méthylène et de méthanol (90-10 en volumes) sous une pression de 0,5 bar (51 kPa) en recueillant des fractions de 100 $cm^3$. Les 12 premières fractions sont éliminées, les 3 fractions suivantes sont réunies et concentrées à sec sous pression réduite (20 mm de mercure; 2,7 kPa) à une température voisine de 45°C. On obtient ainsi 3,7 g de produit brut que l'on dissout dans 20 $cm^3$ d'éthanol. A la solution obtenue, on ajoute 7 $cm^3$ d'une solution éthanolique de gaz chlorhydrique 5N puis 140 $cm^3$ d'éther diéthylique. Le liquide surnageant est éliminé et le précipite huileux est dissous à une température voisine de 50°C dans 60 $cm^3$ d'une solution éthanolique de gaz chlorhydrique 5N. La

solution obtenue est refroidie à une température voisine de 4°C pendant 1 heure. Les cristaux apparus sont séparés par filtration, lavés 2 fois par 10 $cm^3$ au total d'éthanol et 3 fois par 60 $cm^3$ au total d'éther diéthylique puis séchés sous pression réduite (20 mm de mercure; 2,7 kPa) à une température voisine de 40°C en présence de potasse en pastilles. On obtient ainsi 2,65 g de produit fondant à 170°C. 2,4 g de ce produit sont mis en suspension dans 12 $cm^3$ d'une solution éthanolique de gaz chlorhydrique 4,5N. La suspension obtenue est agitée à une température voisine de 20°C pendant 30 minutes. Les cristaux sont séparés par filtration, lavés 2 fois par 6 $cm^3$ au total d'éthanol et 4 fois par 40 $cm^3$ au total d'éther diéthylique puis séchés sous pression réduite (20 mm de mercure; 2,7 kPa) à une température voisine de 20°C en présence de potasse en pastilles. On obtient ainsi 2,7 g de [(méthyl-4 pipérazinyl-1) carbonyl]-7 (pyridyl-3)-3 1H,3H-pyrrolo [1,2-c] thiazole à l'état de trichlorhydrate sous forme de cristaux blancs fondant à 161°C.

L'acide (pyridyl-3)-3 1H,3H-pyrrolo [1,2-c] thiazolecarboxylique-7 est préparé comme à l'exemple 2.

La présente invention concerne également les médicaments constitués par un produit de formule générale (I), sous forme libre ou sous forme de sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale, rectale ou topique.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables.

Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérisilants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les produits selon l'invention sont particulièrement utiles dans le traitement des affections allergiques et inflammatoires et, d'une façon générale, dans toutes les affections dans lesquelles le rôle physiopathologique du PAF-Acéther peut être incriminé.

Les doses dépendent de l'effet recherché et de la durée du traitement; elles sont généralement comprises entre 25 et 100 mg par jour par voie orale, intraveineuse ou par inhalation pour un adulte en une ou plusieurs prises.

D'une façon générale, le médicin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

*Exemple A*

On prépare, selon la technique habituelle, des comprimés dosés à 25 mg de produit actif ayant la composition suivante:

| | |
|---|---|
| -N-Pyridyl-2 (pyridyl-3)-3 1H,3H-pyrrolo [1,2-c] thiazolecarboxamide-7 | 25 mg |
| -amidon | 60 mg |
| -lactose | 50 mg |
| -stéarate de magnésium | 2 mg |

*Exemple B*

On prépare, selon la technique habituelle, une solution injectable contenant 5 mg de produit actif ayant la composition suivante:

| | | |
|---|---|---|
| -(Pyridyl-3)-3 1H,3H-pyrrolo [1,2-c] thiazolecarboxamide-7 | | 5 mg |
| -solution d'acide chlorhydrique 0,1 N | | 0,2 $cm^3$ |
| -soluté injectable | q.s.p. | 2 $cm^3$ |

**Revendications pour les Etats contractants:**
BE, CH, DE, FR, GB, IT, LU, NL, SE

1. Dérivé du 1H,3H-pyrrolo [1,2-c] thiazole, caractérisé en ce qu'il répond à la formule générale:

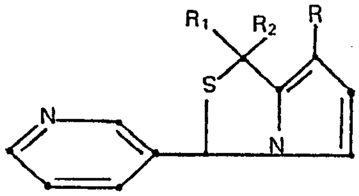

(I)

dans laquelle $R_1$ et $R_2$ identiques ou différents, représentent un atome d'hydrogène ou un radical alcoyle et

a) soit R représente un radical cyano ou alcoylcarbonyle

b) soit R représente un radical de formule générale:

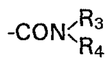

(II)

dans laquelle

— ou bien $R_3$ représente un atome d'hydrogène et $R_4$ représente un radical amino, alcoylamino, dialcoylamino, phénylamino ou diphénylamino,

— ou bien $R_3$ et $R_4$, identiques ou différents, représentent un atome d'hydrogène ou un radical alcoyle contenant 1 à 5 atomes de carbone, ou phényle éventuellement substitué,

— ou bien $R_3$ représente un atome d'hydrogène et $R_4$ représente un radical pyridyle ou alcoyle contenant 1 à 5 atomes de carbone substitué par un radical carboxy, amino, alcoylamino, dialcoylamino, morpholino, pipéridino, pyrrolidinyle-1, pipérazinyle-1 (éventuellement substitué en position 4 par un radical alcoyle, pyridyle, phényle éventuellement substitué ou benzyle éventuellement substitué), phényle éventuellement substitué, pyridyle ou imidazolyle,

— ou bien $R_3$ et $R_4$ forment ensemble un radical imidazolyle ou un hétérocycle à 5 ou 6 chaînons pouvant contenir en outre un autre hétéroatome tel que l'oxygène, le soufre ou l'azote éventuellement substitué par un radical alcoyle, alcoyloxycarbonyle, hydroxyalcoyle, aminoalcoyle, alcoylaminoalcoyle, dialcoylaminoalcoyle, pyridyle, pyrimidinyle, pyrazinyle, phényle, éventuellement substitué ou benzyle éventuellement substitué

c) soit R représente un radical de formule générale:

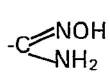

(III) ou

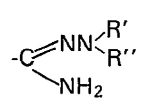

(IV)

dans laquelle R' et R'', identiques ou différents représentent un radical alcoyle,

étant entendu que dans les définitions précédentes les radicaux alcoyle et portions alcoyle sont en chaîne droite ou ramifiée et contiennent, sauf mention spéciale, 1 à 4 atomes de carbone, que les radicaux phényle et benzyle substitués sont des radicaux phényle et benzyle portant un atome d'halogène ou un radical alcoyle, alcoyloxy, alcoylthio, trifluorométhyle ou dialcoylamino, et que l'invention concerne également les formes tautomères des produits cités lorsque R représente un radical de formule générale (III) ou (IV), ainsi que ses sels d'addition avec les acides et, le cas échéant, ses sels métalliques et ses sels d'addition avec les bases azotées.

2. Procédé de préparation d'un produit selon la revendication 1 dans la formule duquel R représente un radical cyano et $R_1$ et $R_2$ sont définis comme à la revendication 1, caractérisé en ce que l'on fait agir le chloro-2 acrylonitrile sur un produit de formule générale:

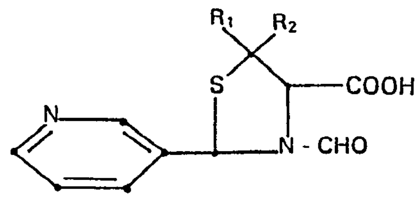

dans laquelle $R_1$ et $R_2$ sont définis comme à la revendication 1 puis isole le produit et le transforme éventuellement en un sel d'addition avec un acide.

3. Procédé de préparation d'un produit selon la revendication 1 dans la formule duquel $R_1$ et $R_2$ sont définis comme à la revendication 1 et R représente un radical de formule générale:

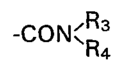

dans laquelle $R_3$ et $R_4$ forment ensemble un cycle imidazolyle, caractérisé en ce que l'on fait agir le N,N'-carbonyldiimidazole sur un acide de formule générale:

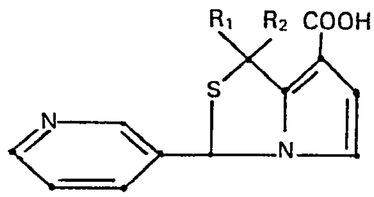

dans laquelle $R_1$ et $R_2$ sont définis comme à la revendication 1, puis isole le produit obtenu et le transforme éventuellement en un sel d'addition avec un acide.

4. Procédé de préparation d'un produit selon la revendication 1 dans la formue duquel $R_1$ et $R_2$ sont définis comme à la revendication 1 et R représente un radical de formule générale:

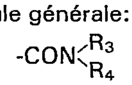

dans laquelle $R_3$ et $R_4$ sont définis comme à la revendication 1 en b) à l'exception pour $R_3$ et $R_4$ de former ensemble un radical imidazolyle, caractérisé en ce que l'on fait réagir l'ammoniac ou un produit de formule générale:

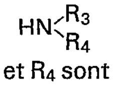

dans laquelle $R_3$ et $R_4$ sont définis comme à la revendication 1 en b) à l'exception pour $R_3$ et $R_4$ de former ensemble un cycle imidazolyle, sur un acide de formule générale:

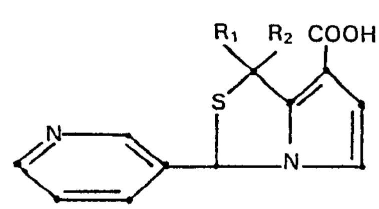

dans laquelle $R_1$ et $R_2$ sont définis comme à la revendication 1, puis isole le produit obtenu et le transforme éventuellement en un sel d'addition avec un acide ou, le cas échéant, en un sel métallique ou un sel d'addition avec une base azotée.

5. Procédé de préparation d'un produit selon la revendication 1 dans la formule duquel $R_1$ et $R_2$ sont définis comme à la revendication 1 et R représente un radical de formule générale:

$$-CON\genfrac{}{}{0pt}{}{R_3}{R_4}$$

dans laquelle $R_3$ et $R_4$ représentent chacun un atome d'hydrogène, caractérisé en ce que l'on hydrolyse un nitrile de formule générale:

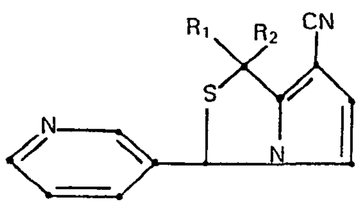

dans laquelle $R_1$ et $R_2$ sont définis comme à la revendication 1, par toute méthode connue de l'homme du métier, puis isole le produit obtenu et le transforme éventuellement en un sel d'addition avec un acide.

6. Procédé de préparation d'un produit selon la revendication 1 dans la formule duqel $R_1$ et $R_2$ sont définis comme à la revendication 1 et R représente un radical de formule générale:

$$-CON\genfrac{}{}{0pt}{}{R_3}{R_4}$$

dans laquelle $R_3$ et $R_4$ représentent chacun un atome d'hydrogène, caractérisé en ce que l'on fait réagir la chloro-2 acrylamide sur un acide de formule générale:

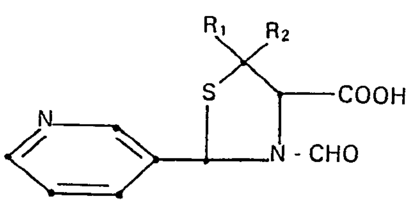

dans laquelle $R_1$ et $R_2$ sont définis comme à la revendication 1, puis isole le produit obtenu et le transforme éventuellement en un sel d'addition avec un acide.

7. Procédé de préparation d'un produit selon la revendication 1 dans la formule générale duquel $R_1$ et $R_2$ sont définis comme à la revendication 1 et R représente un radical acétyle, caractérisé en ce que l'on fait réagir le dérivé éthoxymagnésien du malonate d'éthyle sur un produit de formule générale:

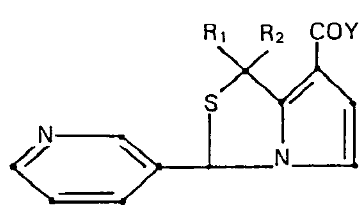

dans laquelle $R_1$ et $R_2$ sont définis comme à la revendication 1 et Y représente un atome d'halogène, puis hydrolyse et décarboxyle le produit obtenu et le transforme éventuellement en un sel d'addition avec un acide.

8. Procédé de préparation d'un produit selon la revendication 1 dans la formule duquel $R_1$ et $R_2$ sont définis comme à la revendication 1 et R représente un radical alcoylcarbonyle, caractérisé en ce que l'on fait réagir un dérivé organomagnésien de formule générale:

$$R''' Mg X$$

dans laquelle R''' représente un radical alcoyle et X représente un atome d'halogène sur un nitrile de formule générale:

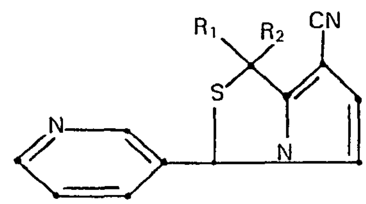

dans laquelle $R_1$ et $R_2$ sont définis comme à la revendication 1, puis hydrolyse le produit obtenu et transforme éventuellement le produit final en un sel d'addition avec un acide.

9. Procédé de préparation d'un produit selon la revendication 1 dans la formule duquel $R_1$ et $R_2$ sont définis comme à la revendication 1 et R représente un radical de formule générale:

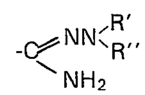

dans laquelle R' et R'' sont définis comme à la revendication 1, caractérisé en ce que l'on fait réagir une hydrazine de formule générale:

$$H_2NN\genfrac{}{}{0pt}{}{R'}{R''}$$

dans laquelle R' et R'' sont définis comme à la revendication 1, sur un produit de formule générale:

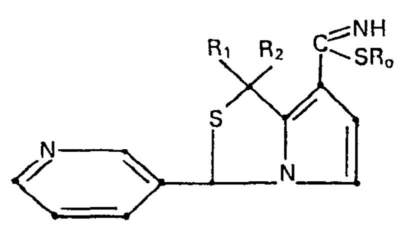

dans laquelle $R_1$ et $R_2$ sont définis comme à la revendication 1 et $R_o$ représente un radical alcoyle, puis isole le produit obtenu et le transforme éventuellement en un sel d'addition avec un acide.

10. Procédé de préparation d'un produit selon la revendication 1 dans la formule duquel $R_1$ et $R_2$ sont définis comme à la revendication 1 et R représente un radical de formule:

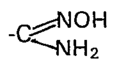

caractérisé en ce que l'on fait agir l'hydroxylamine sur un produit de formule générale:

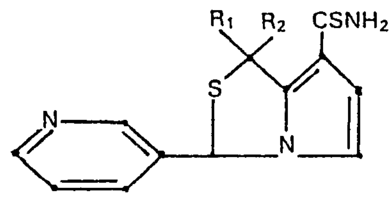

dans laquelle $R_1$ et $R_2$ sont définis comme à la revendication 1, puis isole le produit obtenu et le transforme éventuellement en un sel d'addition avec un acide.

11. Composition pharmaceutique, caractérisé en ce qu'elle contient comme produit actif au moins un composé selon la revendication 1 éventuellement en association avec un ou plusieurs diluants ou adjuvants compatibles et pharmaceutiquement acceptables.

**Revendication pour l'Etat contractant:** AT

1. Procédé de préparation d'un dérivé du 1H,3H-pyrrolo [1,2-c] thiazole de formule générale:

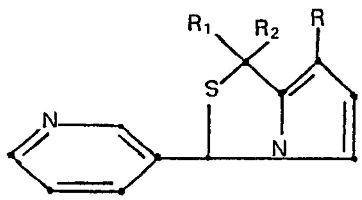

(I)

dans laquelle $R_1$ et $R_2$ identiques ou différents, représentent un atome d'hydrogène ou un radical alcoyle et

a) soit R représente un radical cyano ou alcoylcarbonyle

b) soit R représente un radical de formule générale:

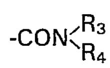

(II)

dans laquelle

— ou bien $R_3$ représente un atome d'hydrogène et $R_4$ représente un radical amino, alcoylamino, dialcoylamino, phénylamino ou diphénylamino,

— ou bien $R_3$ et $R_4$, identiques ou différents, représentent un atome d'hydrogène ou un radical alcoyle contenant 1 à 5 atomes de carbone, ou phényle éventuellement substitué,

— ou bien $R_3$ représente un atome d'hydrogène et $R_4$ représente un radical pyridyle ou alcoyle contenant 1 à 5 atomes de carbone substitué par un radical carboxy, amino, alcoylamino, dialcoylamino, morpholino, pipéridino, pyrrolidinyle-1, pipérazinyle-1 (éventuellement substitué en position 4 par un radical alcoyle, pyridyle, phényle éventuellement substitué ou benzyle éventuellement substitué), phényle éventuellement substitué, pyridyle ou imidazolyle,

— ou bien $R_3$ et $R_4$ forment ensemble un radical imidazolyle ou un hétérocycle à 5 ou 6 chaînons pouvant contenir en outre un autre hétéroatome tel que l'oxygène, le soufre ou l'azote éventuellement substitué par un radical alcoyle, alcoyloxycarbonyle, hydroxyalcoyle, aminoalcoyle, alcoylaminoalcoyle, dialcoylaminoalcoyle, pyridyle, pyrimidinyle, pyrazinyle, phényle, éventuellement substitué ou benzyle éventuellement substitué

c) soit R représente un radical de formule générale:

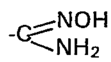

(III) ou

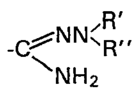

(IV)

dans laquelle R' et R'', identiques ou différents représentent un radical alcoyle,

étant entendu que dans les définitions précédentes les radicaux alcoyle et portions alcoyle sont en chaîne droite ou ramifiée et contiennent, sauf mention spéciale, 1 à 4 atomes de carbone, que les radicaux phényle et benzyle substitués sont les radicaux phényle et benzyle portant un atome d'halogène ou un radical alcoyle, alcoyloxy, alcoylthio, trifluorométhyle ou dialcoylamino, et que l'invention concerne également les formes tautomères des produits cités lorsque R représente un radical de formule générale (III) ou (IV), ainsi que ses sels d'addition avec les acides et, le cas échéant, ses sels métalliques et ses sels d'addition avec les bases azotées, caractérisé en ce que

A - pour la préparation d'un produit de formule générale (I) dans laquelle R représente un radical cyano et $R_1$ et $R_2$ sont définis comme précédemment, l'on fait agir le chloro-2 acrylonitrile sur un produit de formule générale:

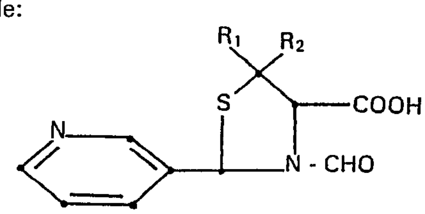

dans laquelle $R_1$ et $R_2$ sont définis comme précédemment puis isole le produit et le transforme éventuellement en un sel d'addition avec un acide, ou en ce que

B - pour la préparation d'un produit de formule générale (I) dans laquelle $R_1$ et $R_2$ sont définis comme précédemment et R représente un radical de formule générale:

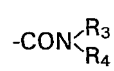

dans laquelle $R_3$ et $R_4$ forment ensemble un cycle imidazolyle, l'on fait agir le N,N'-carbonyldiimidazole sur un acide de formule générale:

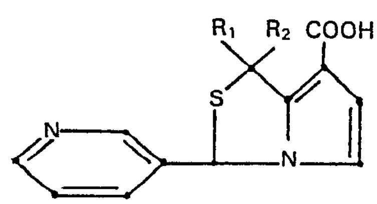

dans laquelle $R_1$ et $R_2$ sont définis comme précédemment, puis isole le produit obtenu et le transforme éventuellement en un sel d'addition avec un acide, ou en ce que

C - pour la préparation d'un produit de formule générale (I) dans laquelle $R_1$ et $R_2$ sont définis comme précédemment et R représente un radical de formule générale:

$$-CON{<}^{R_3}_{R_4}$$

dans laquelle $R_3$ et $R_4$ sont définis comme précédemment en b) à l'exception pour $R_3$ et $R_4$ de former ensemble un radical imidazolyle, l'on fait réagir l'ammoniac ou un produit de formule générale:

$$HN{<}^{R_3}_{R_4}$$

dans laquelle $R_3$ et $R_4$ sont définis comme précédemment en b) à l'exception pour $R_3$ et $R_4$ de former ensemble un cycle imidazolyle, sur un acide de formule générale:

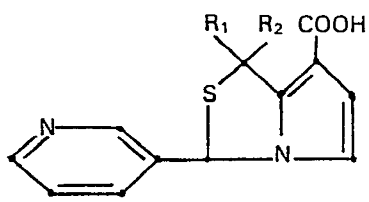

dans laquelle $R_1$ et $R_2$ sont définis comme précédemment, puis isole le produit obtenu et le transforme éventuellement en un sel d'addition avec un acide ou, le cas échéant, en un sel métallique ou un sel d'addition avec une base azotée, ou en ce que

D - pour la préparation d'un produit de formule générale (I) dans laquelle $R_1$ et $R_2$ sont définis comme précédemment et R représente un radical de formule générale:

$$-CON{<}^{R_3}_{R_4}$$

dans laquelle $R_3$ et $R_4$ représentent chacun un atome d'hydrogène, l'on hydrolyse un nitrile de formule générale:

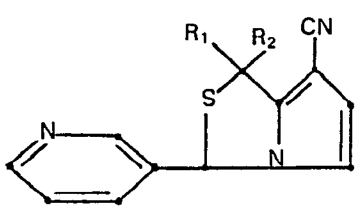

dans laquelle $R_1$ et $R_2$ sont définis comme précédemment par toute méthode connue de l'homme du métier, puis isole le produit obtenu et le transforme éventuellement en un sel d'addition avec un acide, ou en ce que

E - pour la préparation d'un produit de formule générale (I) dans laquelle $R_1$ et $R_2$ sont définis comme précédemment et R représente un radical de formule générale:

$$-CON{<}^{R_3}_{R_4}$$

dans laquelle $R_3$ et $R_4$ représentent chaun un atome d'hydrogène, l'on fait réagir la chloro-2 acrylamide sur un acide de formule générale:

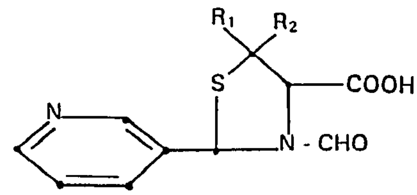

dans laquelle $R_1$ et $R_2$ sont définis comme précédemment, puis isole le produit obtenu et le transforme éventuellement en un sel d'addition avec un acide, ou en ce que

F - pour la préparation d'un produit de formule générale (I) dans laquelle $R_1$ et $R_2$ sont définis comme précédemment et R représente un radical acétyle, l'on fait réagir le dérivé éthoxymagnésien du malonate d'éthyle sur un produit de formule générale:

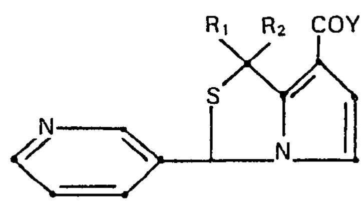

dans laquelle $R_1$ et $R_2$ sont définis comme précédemment, et Y représente un atome d'halogène, puis hydrolyse et décarboxyle le produit obtenu et le transforme éventuellement en un sel d'addition avec un acide, ou en ce que

G - pour la préparation d'un produit de formule générale (I) dans laquelle $R_1$ et $R_2$ sont définis comme précédemment et R représente un radical alcoylcarbonyle, l'on fait réagir un dérivé organomagnésien de formule générale:

$$R''' \ Mg \ X$$

dans laquelle R''' représente un radical alcoyle et X représente un atome d'halogene sur un nitrile de formule générale:

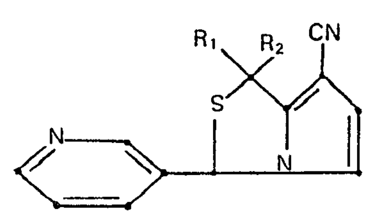

dans laquelle $R_1$ et $R_2$ sont définis comme précédemment, puis hydrolyse le produit obtenu et transforme éventuellement le produit final en un sel d'addition avec un acide, ou en ce que

H - pour la préparation d'un produit de formule générale (I) dans laquelle $R_1$ et $R_2$ sont définis comme précédemment et R représente un radical de formule générale:

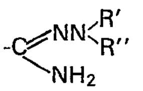

dans laquelle R' et R'' sont définis comme précédemment, l'on fait réagir une hydrazine de formule générale:

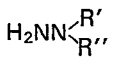

dans laquelle R' et R'' sont définis comme précédemment sur un produit de formule générale:

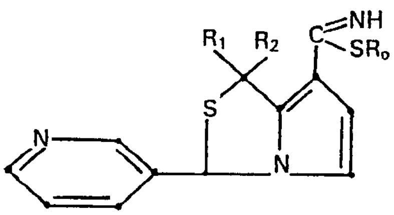

dans laquelle $R_1$ et $R_2$ sont définis comme précédemment et $R_0$ représente un radical alcoyle, puis isole le produit obtenu et le transforme éventuellement en un sel d'addition avec un acide, ou en ce que I - pour la préparation d'un produit de formule générale (I) dans laquelle $R_1$ et $R_2$ sont définis comme précédemment et R représente un radical de formule:

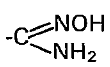

l'on fait agir l'hydroxylamine sur un produit de formule générale:

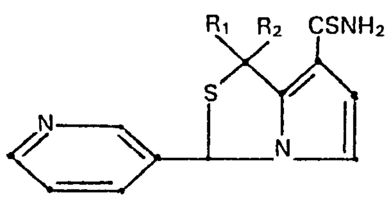

dans laquelle $R_1$ et $R_2$ sont définis comme précédemment, puis isole le produit obtenu et le transforme éventuellement en un sel d'addition avec un acide.

**Patentansprüche für die Vertragsstaaten:**
BE, CH, DE, FR, GB, IT, LU, NL, SE

1. Derivat des 1H,3H-Pyrrolo[1,2-c]thiazols, dadurch gekennzeichnet, dass es der allgemeinen Formel

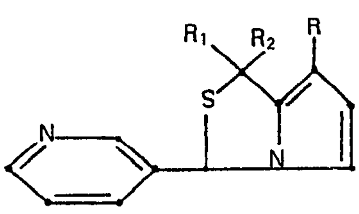

(I)

entspricht, worin $R_1$ und $R_2$, die identisch oder verschieden sind, ein Wasserstoffatom oder einen Alkylrest bedeuten, und

a) entweder R bedeutet einen Cyano- oder Alkylcarbonylrest, oder

b) R bedeutet einen Rest der allgemeinen Formel

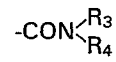

(II)

worin

— entweder $R_3$ ein Wasserstoffatom bedeutet und $R_4$ einen Amino-, Alkylamino-, Dialkylamino-, Phenylamino- oder Diphenylaminorest bedeutet,

— oder $R_3$ und $R_4$, die identisch oder verschieden sind, bedeuten ein Wasserstoffatom oder einen Alkylrest mit 1 bis 5 Kohlenstoffatomen oder gegebenenfalls substituiertes Phenyl,

— oder $R_3$ bedeutet ein Wasserstoffatom und $R_4$ bedeutet einen Pyridyl- oder Alkylrest mit 1 bis 5 Kohlenstoffatomen, substituiert durch einen Carboxy-, Amino-, Alkylamino-, Dialkylamino-, Morpholino-, Piperidino-, Pyrrolidinyl-1-, Piperazinyl-1 (gegebenenfalls substituiert in 4-Stellung durch einen Alkyl-, Pyridyl-, gegebenenfalls substituierten Phenyl- oder gegebenenfalls substituierten Benzylrest), gegebenenfalls substituierten Phenyl-, Pyridyl- oder Imidazolylrest,

— oder $R_3$ und $R_4$ bilden zusammen einen Imidazolylrest oder einen Heterocyclus mit 5 oder 6 Kettengliedern, der ausserdem ein anderes Heteroatom enthalten kann wie Sauerstoff, Schwefel oder Stickstoff, gegebenenfalls substituiert durch einen Alkyl-, Alkyloxycarbonyl-, Hydroxyalkyl-, Aminoalkyl-, Alkylaminoalkyl-, Dialkylaminoalkyl-, Pyridyl-, Pyrimidinyl-, Pyrazinyl-, gegebenenfalls substituierten Phenyl- oder gegebenenfalls substituierten Benzylrest, oder

c) R bedeutet einen Rest der allgemeinen Formel

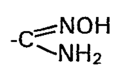 (III) oder 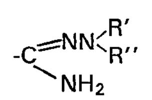 (IV)

worin R' und R'', die identisch oder verschieden sind, einen Alkylrest bedeuten,
wobei in den vorstehenden Definitionen die Alkylreste und Alkylteile in gerader oder verzweigter Kette sind und wenn nichts anderes angegeben ist, 1 bis 4 Kohlenstoffatome enthalten, wobei die substituierten Phenyl- und Benzylreste, die Phenyl- und Benzylreste sind, die ein Halogenatom oder einen Alkyl-, Alkyloxi-, Alkylthio-, Trifluormethyl- oder Dialkylaminorest tragen, und wobei die Erfindung auch die tautomeren Formen der erwähnten Produkte umfasst, wenn R einen Rest der allgemeinen Formel (III) oder (IV) bedeutet, sowie seine Additionssalze mit Säuren und gegebenenfalls seine Metallsalze und seine Additionssalze mit Stickstoffbasen.

2. Verfahren zur Herstellung eines Produkts gemäss Anspruch 1, in dessen Formel R einen Cyanorest bedeutet, und $R_1$ und $R_2$ wie in Anspruch 1 definiert sind, dadurch gekennzeichnet, dass man 2-Chlor-acrylnitril auf ein Produkt der allgemeinen Formel

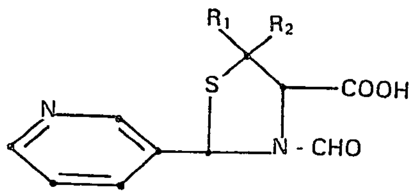

einwirken lässt, worin $R_1$ und $R_2$ wie in Anspruch 1 definiert sind, dass man das erhaltene Produkt dann isoliert und es gegebenenfalls in ein Additionssalz mit einer Säure überführt.

3. Verfahren zur Herstellung eines Produkts gemäss Anspruch 1, in dessen Formel $R_1$ und $R_2$ wie in Anspruch 1 definiert sind, und R einen Rest der allgemeinen Formel

$$-CON\begin{matrix} R_3 \\ R_4 \end{matrix}$$

bedeutet, worin $R_3$ und $R_4$ zusammen einen Imidazolylring bilden, dadurch gekennzeichnet, dass man N,N'-Carbonyldiimidazol auf eine Säure der allgemeinen Formel

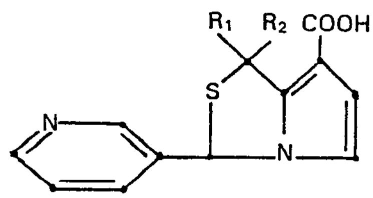

worin $R_1$ und $R_2$ wie in Anspruch 1 definiert sind, einwirken lässt, dass man das erhaltene Produkt dann isoliert und es gegebenenfalls in ein Additionssalz mit einer Säure überführt.

4. Verfahren zur Herstellung eines Produkts gemäss Anspruch 1, in dessen Formel $R_1$ und $R_2$ wie in Anspruch 1 definiert sind, und R einen Rest der allgemeinen Formel

$$-CON\begin{matrix} R_3 \\ R_4 \end{matrix}$$

bedeutet, worin $R_3$ und $R_4$ wie in Anspruch 1 unter b) definiert sind, mit der Ausnahme, dass $R_3$ und $R_4$ zusammen einen Imidazolylrest bilden, dadurch gekennzeichnet, dass man Ammoniak oder ein Produkt der allgemeinen Formel

$$HN\begin{matrix} R_3 \\ R_4 \end{matrix}$$

worin $R_3$ und $R_4$ wie in Anspruch 1 unter b) definiert sind, mit der Ausnahme, dass $R_3$ und $R_4$ zusammen einen Imidazolylring bilden, auf eine Säure der allgemeinen Formel

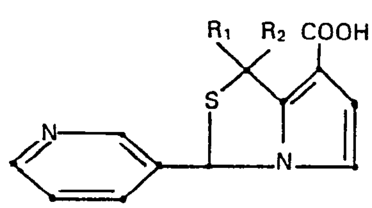

worin $R_1$ und $R_2$ wie in Anspruch 1 definiert sind, einwirken lässt, dass man das erhaltene Produkt dann isoliert und es gegebenenfalls in ein Additionssalz mit einer Säure, oder gegebenenfalls in ein Metallsalz oder ein Additionssalz mit einer Stickstoffbase überführt.

5. Verfahren zur Herstellung eines Produkts gemäss Anspruch 1, in dessen Formel $R_1$ und $R_2$ wie in Anspruch 1 definiert sind, und R einen Rest der allgemeinen Formel

$$-CON\begin{matrix} R_3 \\ R_4 \end{matrix}$$

bedeutet, worin $R_3$ und $R_4$ jeweils ein Wasserstoffatom bedeuten, dadurch gekennzeichnet, dass man ein Nitril der allgemeinen Formel

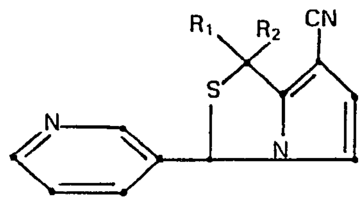

worin $R_1$ und $R_2$ wie in Anspruch 1 definiert sind, durch jede dem Fachmann bekannte Methode hydrolisiert, das erhaltene Produkt dann isoliert und es gegebenenfalls in ein Additionssalz mit einer Säure überführt.

6. Verfahren zur Herstellung eines Produkts gemäss Anspruch 1, in dessen Formel $R_1$ und $R_2$ wie in Anspruch 1 definiert sind, und R einen Rest der allgemeinen Formel

$$-CON\begin{matrix} R_3 \\ R_4 \end{matrix}$$

bedeutet, worin $R_3$ und $R_4$ jeweils ein Wasserstoffatom bedeuten, dadurch gekennzeichnet, dass man 2-Chlor-acrylamid auf eine Säure der allgemeinen Formel

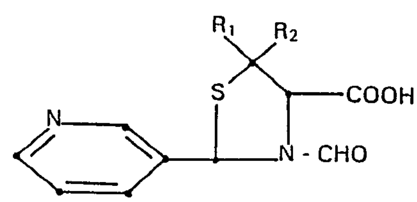

worin $R_1$ und $R_2$ wie in Anspruch 1 definiert sind, einwirken lässt, das erhaltene Produkt dann isoliert und es gegebenenfalls in ein Additionssalz mit einer Säure überführt.

7. Verfahren zur Herstellung eines Produkts gemäss Anspruch 1, in dessen allgemeiner Formel $R_1$ und $R_2$ wie in Anspruch 1 definiert sind, und R einen Acetylrest bedeutet, dadurch gekennzeichnet, dass man das Ethoximagnesiumderivat des Ethylmalonats auf ein Produkt der allgemeinen Formel

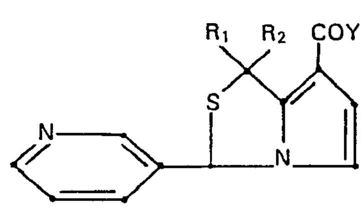

einwirken lässt, worin $R_1$ und $R_2$ wie in Anspruch 1 definiert sind, und Y ein Halogenatom bedeutet, das erhaltene Produkte dann hydrolysiert und decarboxyliert und es gegebenenfalls in ein Additionssalz mit einer Säure überführt.

8. Verfahren zur Herstellung eines Produkts gemäss Anspruch 1, in dessen Formel $R_1$ und $R_2$ wie in Anspruch 1 definiert sind, und R einen Alkylcarbonylrest bedeutet, dadurch gekennzeichnet, dass man ein Organomagnesiumderivat der allgemeinen Formel

$$R''' \, Mg \, X$$

worin R''' einen Alkylrest und X ein Halogenatom bedeutet, auf ein Nitril der allgemeinen Formel

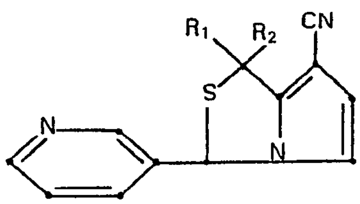

worin $R_1$ und $R_2$ wie in Anspruch 1 definiert sind, einwirken lässt, das erhaltene Produkt dann hydrolysiert, und das Endprodukt gegebenenfalls in ein Additionssalz mit einer Säure überführt.

9. Verfahren zur Herstellung eines Produkts gemäss Anspruch 1, in dessen Formel $R_1$ und $R_2$ wie in Anspruch 1 definiert sind, und R einen Rest der allgemeinen Formel

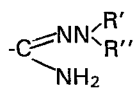

bedeutet, worin R' und R'' wie in Anspruch 1 definiert sind, dadurch gekennzeichnet, dass man ein Hydrazin der allgemeinen Formel

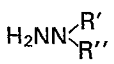

worin R' und R'' wie in Anspruch 1 definiert sind, auf ein Produkt der allgemeinen Formel

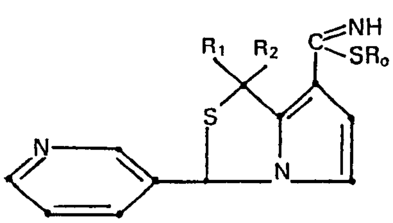

einwirken lässt, worin $R_1$ und $R_2$ wie in Anspruch 1 definiert sind, und $R_0$ einen Alkylrest bedeutet, das erhaltene Produkt dann isoliert und es gegebenenfalls in ein Additionssalz mit einer Säure überführt.

10. Verfahren zur Herstellung eines Produkts gemäss Anspruch 1, in dessen Formel $R_1$ und $R_2$ wie in Anspruch 1 definiert sind, und R einen Rest der Formel

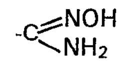

bedeutet, dadurch gekennzeichnet, dass man Hydroxylamin auf ein Produkt der allgemeinen Formel

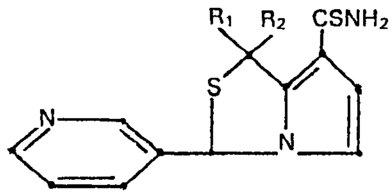

worin $R_1$ und $R_2$ wie in Anspruch 1 definiert sind, einwirken lässt, das erhaltene Produkt dann isoliert und gegebenenfalls in ein Additionssalz in einer Säure überführt.

11. Pharmazeutische Zusammensetzung, dadurch gekennzeichnet, dass sie als aktives Produkt wenigstens eine Verbindung gemäss Anspruch 1 enthält, gegebenenfalls zusammen mit einem oder mehreren verträglichen und pharmazeutisch annehmbaren Verdünnungs- oder Hilfsmitteln.

**Patentanspruch für den Vertragsstaat:** AT

1. Verfahren zur Herstellung eines 1H,3H-Pyrrolo-[1,2-c]thiazol-derivats der allgemeinen Formel

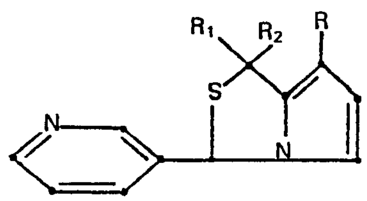

(I)

in welcher $R_1$ und $R_2$ unabhängig voneinander ein Wasserstoffatom oder einen Alkylrest darstellen und

a) R entweder einen Cyano- oder Alkylcarbonylrest darstellt,

b) oder R einen Rest der allgemeinen Formel

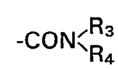

(II)

darstellt, in welcher

— $R_3$ ein Wasserstoffatom darstellt und $R_4$ einen Amino-, Alkylamino-, Dialkylamino-, Phenylamino- oder Diphenylaminorest darstellt,

— oder $R_3$ und $R_4$, unabhängig voneinander ein Wasserstoffatom oder einen Alkylrest mit 1 bis 5 Kohlenstoffatomen oder einen gegebenenfalls substituierten Phenylrest darstellen,

— oder $R_3$ ein Wasserstoffatom darstellt und $R_4$ einen Pyridylrest oder Alkylrest mit 1 bis 5 Kohlenstoffatomen, substituiert durch einen Carboxy-, Amino-, Alkylamino-, Dialkylamino-, Morpholino-, Piperidino-, 1-Pyrrolidinyl-, 1-Piperazinyl-, (gegebenenfalls substituiert in 4-Stellung durch einen Alkyl-, Pyridyl-, gegebenenfalls substituierten Phenyl- oder gegebenenfalls substituierten Benzylrest), gegebenenfalls substituierten Phenyl-, einen Pyridyl- oder Imidazolylrest darstellt,

— oder $R_3$ und $R_4$ zusammen einen Imidazolylrest oder einen Heterocyclus mit 5 oder 6 Gliedern bilden, der ausserdem ein weiteres Heteroatom, z.B. Sauerstoff, Schwefel oder Stickstoff, enthalten kann und gegebenenfalls substituiert sein kann durch einen Alkyl-, Alkyloxycarbonyl-, Hydroxyalkyl-, Aminoalkyl-, Alkylaminoalkyl-, Dialkylaminoalkyl-, Pyridyl-, Pyrimidinyl-, Pyrazinyl-, einen gegebenenfalls substituierten Phenyl- oder einen gegebenenfalls substituierten Benzylrest,

c) oder R einen Rest der allgemeinen Formel

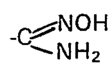 (III) oder 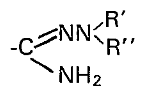 (IV)

darstellt, worin R' und R'' unabhängig voneinander einen Alkylrest darstellen,

wobei die Alkylreste und Alkylteile in den obigen Definitionen selbstverständlich geradkettig oder verzweigt sind und ohne besonderen Hinweis 1 bis 4 Kohlenstoffatome enthalten, die substituierten Phenyl- und Benzylreste Phenyl- und Benzylreste sind, die ein Halogenatom oder einen Alkyl-, Alkyloxy-, Alkylthio-, Trifluormethyl- oder Dialkylaminorest tragen, und die Erfindung auch die tautomeren Formen der erwähnten Verbindungen umfasst, wenn R einen Rest der allgemeinen Formel (III) oder (IV) darstellt, sowie von seinen Säureadditionssalzen und zutreffendenfalls seinen Metallsalzen und Additionssalzen mit Stickstoffbasen, dadurch gekennzeichnet, dass man

A - zur Herstellung einer Verbindung der allgemeinen Formel (I), in welcher R einen Cyanorest darstellt und $R_1$ und $R_2$ die obige Bedeutung haben, 2-Chlor-acrylnitril mit einer Verbindung der allgemeinen Formel

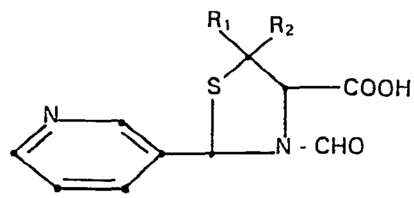

in welcher $R_1$ und $R_2$ die obige Bedeutung haben, umsetzt, dann die Verbindung isoliert und gegebenenfalls in ein Säureadditionssalz überführt, oder dass man

B - zur Herstellung einer Verbindung der allgemeinen Formel (I), in welcher $R_1$ und $R_2$ die obige Bedeutung haben und R einen Rest der allgemeinen Formel

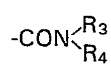

darstellt, in welcher $R_3$ und $R_4$ zusammen einen Imidazolylring bilden, N,N'-Carbonyldiimidazol mit einer Säure der allgemeinen Formel

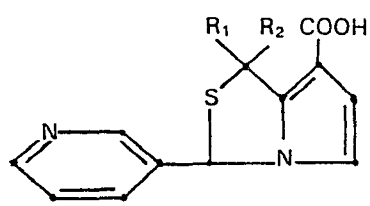

in welcher $R_1$ und $R_2$ die obige Bedeutung haben, umsetzt, dann die erhaltene Verbindung isoliert und sie gegebenenfalls in ein Säureadditionssalz überführt, oder dass man

C - zur Herstellung einer Verbindung der allgemeinen Formel (I), in welcher $R_1$ und $R_2$ die obige Bedeutung haben und R einen Rest der allgemeinen Formel

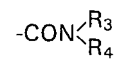

darstellt, in welcher $R_3$ und $R_4$ die oben unter b) angegebene Bedeutung, mit der Ausnahme, dass $R_3$ und $R_4$ zusammen einen Imidazolylrest bilden, haben, Ammoniak oder eine Verbindung der allgemeinen Formel

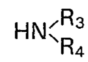

in welcher $R_3$ und $R_4$ die oben unter b) angegebene Bedeutung, mit der Ausnahme, dass $R_3$ und $R_4$ zusammen einen Imidazolylrest bilden, haben, mit einer Säure der allgemeinen Formel

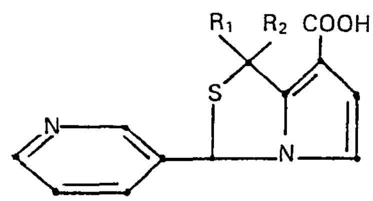

in welcher $R_1$ und $R_2$ die obige Bedeutung haben, umsetzt, dann die erhaltene Verbindung isoliert und sie gegebenenfalls in ein Säureadditionssalz oder zutreffendenfalls in ein Metallsalz oder in ein Additionssalz mit einer Stickstoffbase umwandelt, oder dass man

D - zur Herstellung einer Verbindung der allgemeinen Formel (I), in welcher $R_1$ und $R_2$ die obige Bedeutung haben und R einen Rest der allgemeinen Formel

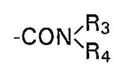

darstellt, in welcher $R_3$ und $R_4$ jeweils ein Wasserstoffatom darstellen, ein Nitril der allgemeinen Formel

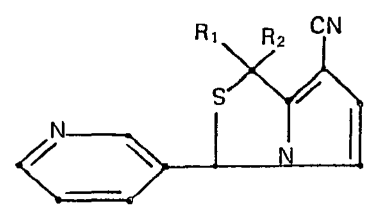

in welcher $R_1$ und $R_2$ die obige Bedeutung haben, nach irgendeiner dem Fachmann bekannten Methode hydrolysiert, dann die erhaltene Verbindung isoliert und sie gegebenenfalls in ein Säureadditionssalz umwandelt, oder dass man

E - zur Herstellung einer Verbindung der allgemeinen Formel (I), in welcher $R_1$ und $R_2$ die obige Bedeutung haben und R einen Rest der allgemeinen Formel

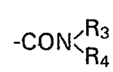

darstellt, in welcher $R_3$ und $R_4$ jeweils ein Wasserstoffatom darstellen, 2-Chlor-acrylamid mit einer Säure der allgemeinen Formel

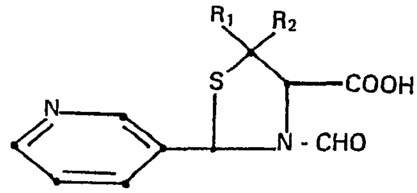

in welcher $R_1$ und $R_2$ die obige Bedeutung haben, umsetzt, dann die erhaltene Verbindung isoliert und sie gegebenenfalls in ein Säureadditionssalz umwandelt, oder dass man

F - zur Herstellung einer Verbindung der allgemeinen Formel (I), in welcher $R_1$ und $R_2$ die obige Bedeutung haben und R einen Acetylrest darstellt, das Äthoxymagnesiumderivat von Äthylmalonat mit einer Verbindung der allgemeinen Formel

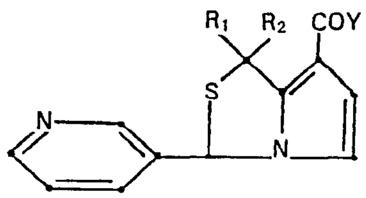

in welcher $R_1$ und $R_2$ die obige Bedeutung haben und Y ein Halogenatom darstellt, umsetzt, dann die erhaltene Verbindung hydrolysiert und decarboxyliert und sie gegebenenfalls in ein Säureadditionssalz umwandelt, oder dass man

G - zur Herstellung einer Verbindung der allgemeinen Formel (I), in welcher $R_1$ und $R_2$ die obige Bedeutung haben und R einen Alkylcarbonylrest darstellt, ein Organomagnesiumderivat der allgemeinen Formel

$$R''' \; Mg \; X,$$

in welcher R''' einen Alkylrest darstellt und X ein Halogenatom darstellt, mit einem Nitril der allgemeinen Formel

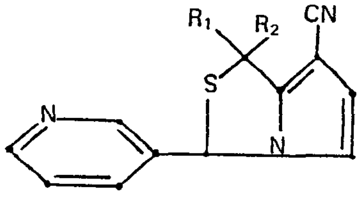

in welcher $R_1$ und $R_2$ die obige Bedeutung haben, umsetzt, dann die erhaltene Verbindung hydrolysiert und das Endprodukt gegebenenfalls in ein Säureadditionssalz umwandelt, oder dass man

H - zur Herstellung einer Verbindung der allgemeinen Formel (I), in welcher $R_1$ und $R_2$ die obige Bedeutung haben und R einen Rest der allgemeinen Formel

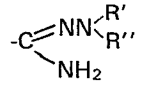

in welcher R' und R'' die obige Bedeutung haben, darstellt, ein Hydrazin der allgemeinen Formel

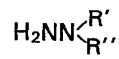

in welcher R' und R'' die obige Bedeutung haben, mit einer Verbindung der allgemeinen Formel

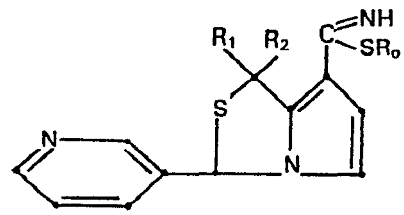

in welcher $R_1$ und $R_2$ die obige Bedeutung haben und $R_o$ einen Alkylrest darstellt, umsetzt, dann die erhaltene Verbindung isoliert und sie gegebenenfalls in ein Säureadditionssalz umwandelt, oder dass man

I - zur Herstellung einer Verbindung der allgemeinen Formel (I), in welcher $R_1$ und $R_2$ die obige Bedeutung haben und R einen Rest der Formel

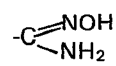

darstellt, Hydroxylamin mit einer Verbindung der allgemeinen Formel

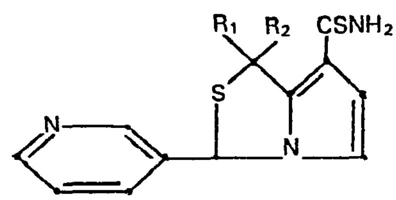

in welcher $R_1$ und $R_2$ die obige Bedeutung haben, umsetzt, dann die erhaltene Verbindung isoliert und sie gegebenenfalls in ein Säureadditionssalz umwandelt.

**Claims for the contracting states:**
BE, CH, DE, FR, GB, IT, LU, NL, SE

1. 1H,3H-pyrrolo[1,2-c]thiazole derivative, characterized in that it corresponds to the general formula:

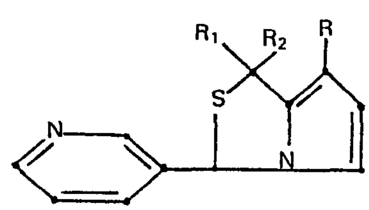

(I)

in which $R_1$ and $R_2$, which may be identical or different, denote a hydrogen atom or an alkyl radical and

a) either R denotes a cyano or alkylcarbonyl radical,

b) or R denotes a radical of general formula:

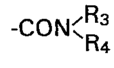

(II)

in which

— either $R_3$ denotes a hydrogen atom and $R_4$ denotes an amino, alkylamino, dialkylamino, phenylamino or diphenylamino radical,

— or $R_3$ and $R_4$, which may be identical or different, denote a hydrogen atom or an alkyl radical containing 1 to 5 carbon atoms or an optionally substituted radical,

— or $R_3$ denotes a hydrogen atom and $R_4$ denotes a pyridyl radical or an alkyl radical containing 1 to 5 carbon atoms, substituted with a carboxy, amino, alkylamino, dialkylamino, morpholino, piperidino or 1-pyrrolidinyl radical, a 1-piperazinyl radical (optionally substituted at position 4 with an alkyl or pyridyl radical or an optionally substituted phenyl or optionally substituted benzyl radical), an optionally substituted phenyl radical or a pyridyl or imidazolyl radical,

or $R_3$ and $R_4$ together form an imidazolyl radical or a 5- or 6-membered heterocyclic ring which can contain in addition another hetero atom such as oxygen, sulphur or nitrogen, and is optionally substituted with an alkyl, alkyloxycarbonyl, hydroxyalkyl, aminoalkyl, alkylaminoalkyl, dialkylaminoalkyl, pyridyl, pyrimidinyl or pyrazinyl radical, or an optionally substituted phenyl or optionally substituted benzyl radical,

c) or R denotes a radical of general formula:

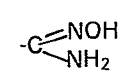 (III) or 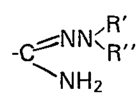 (IV)

in which R' and R'', which may be idendical or different, denote an alkyl radical,

with the understanding that, in the above definitions, the alkyl radicals and alkyl portions are straight-chain or branched and contain, except where otherwise stated, 1 to 4 carbon atoms, that the substituted phenyl and benzyl radicals are phenyl and benzyl radicals bearing a halogen atom or an alkyl, alkyloxy, alkylthio, trifluoromethyl or dialkylamino radical, and that the invention also relates to the tautomeric forms of the abovementioned products when R denotes a radical of general formula (III) or (IV), as well as its addition salts with acids and, where appropriate, its metal salts and addition salts with nitrogenous bases.

2. Process for preparing a product according to Claim 1 in the formula of which R denotes a cyano radical and $R_1$ and $R_2$ are defined as in Claim 1, characterized in that 2-chloroacrylonitrile is reacted with a product of general formula:

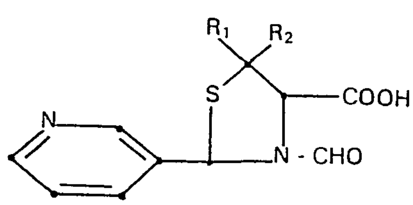

in which $R_1$ and $R_2$ are defined as in Claim 1, and the product is then isolated and optionally converted to an addition salt with an acid.

3. Process for preparing a product according to Claim 1 in the formula of which $R_1$ and $R_2$ are defined as in Claim 1 and R denotes a radical of general formula:

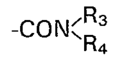

in which $R_3$ and $R_4$ together form an imidazolyl ring, characterized in that N,N'-carbonyldiimidazole is reacted with an acid of general formula:

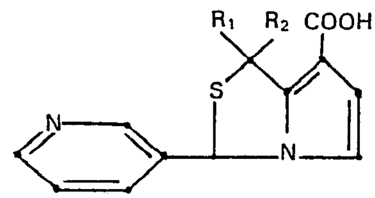

in which $R_1$ and $R_2$ are defined as in Claim 1, and the product obtained is then isolated and optionally converted to an addition salt with an acid.

4. Process for preparing a product according to Claim 1 in the formula of which $R_1$ and $R_2$ are defined as in Claim 1 and R denotes a radical of general formula:

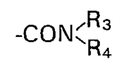

in which $R_3$ and $R_4$ are defined as in Claim 1 at b), with the exception of the possibility of $R_3$ and $R_4$ together forming an imidazolyl radical, characterized in that ammonia or a product of general formula:

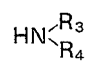

in which $R_3$ and $R_4$ are defined as in Claim 1 at b), with the exception of the possibility of $R_3$ and $R_4$ together forming an imidazolyl ring, is reacted with an acid of general formula:

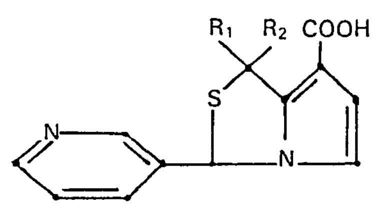

in which $R_1$ and $R_2$ are defined as in Claim 1, and the product obtained is then isolated and optionally converted to an addition salt with an acid or, where appropriate to a metal salt or an addition salt with a nitrogenous base.

5. Process for preparing a product according to Claim 1 in the formula of which $R_1$ and $R_2$ are defined as in Claim 1 and R denotes a radical of general formula:

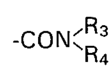

in which $R_3$ and $R_4$ each denote a hydrogen atom, characterized in that a nitrile of general formula:

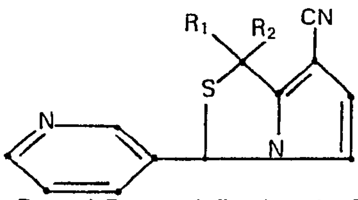

in which $R_1$ and $R_2$ are defined as in Claim 1, is hydrolysed by any method known to those versed in the art, and the product obtained is then isolated and optionally converted to an addition salt with an acid.

6. Process for preparing a product according to Claim 1 in the formula of which $R_1$ and $R_2$ are defined as in Claim 1 and R denotes a radical of general formula:

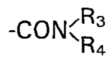

in which $R_3$ and $R_4$ each denote a hydrogen atom, characterized in that 2-chloroacrylamide is reacted with an acid of general formula:

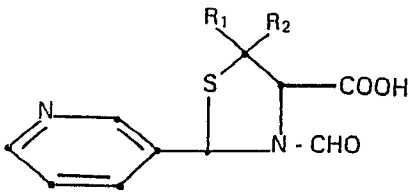

in which $R_1$ and $R_2$ are defined as in Claim 1, and the product obtained is then isolated and optionally converted to an addition salt with an acid.

7. Process for preparing a product according to Claim 1 in the general formula of which $R_1$ and $R_2$ are defined as in Claim 1 and R denotes an acetyl radical, characterized in that the ethoxymagnesium derivative of ethyl malonate is reacted with a product of general formula:

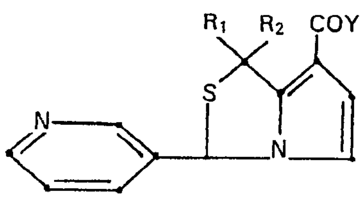

in which $R_1$ and $R_2$ are defined as in Claim 1 and Y denotes a haloten atom, and the product obtained is then hydrolysed and decarboxylated and optionally converted to an addition salt with an acid.

8. Process for preparing a product according to Claim 1 in the formula of which $R_1$ and $R_2$ are defined as in Claim 1 and R denotes an alkylcarbonyl radical, characterized in that an organomagnesium derivative of general formula:

R''' Mg X'

in which R''' denotes an alkyl radical and X denotes a halogen atom, is reacted with a nitrile of general formula:

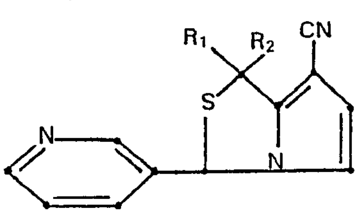

in which $R_1$ and $R_2$ are defined as in Claim 1, and the product obtained is then hydrolysed and the final product optionally converted to an addition salt with an acid.

9. Process for preparing a product according to Claim 1 in the formula of which $R_1$ and $R_2$ are defined as in Claim 1 and R denotes a radical of general formula:

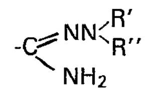

in which R' and R'' are defined as in Claim 1, characterized in that a hydrazine of general formula:

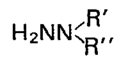

in which R' and R'' are defined as in Claim 1, is reacted with a product of general formula:

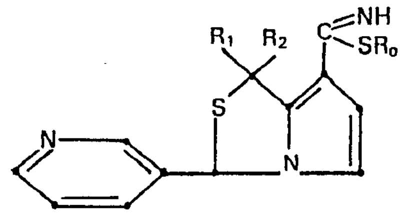

in which $R_1$ and $R_2$ are defined as in Claim 1 and $R_o$ denotes an alkyl radical, and the product obtained is then isolated and optionally converted to an addition salt with an acid.

10. Process for preparing a product according to Claim 1 in the formula of which $R_1$ and $R_2$ are defined as in Claim 1 and R denotes a radical of formula:

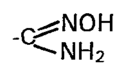

characterized in that hydroxylamine is reacted with a product of general formula:

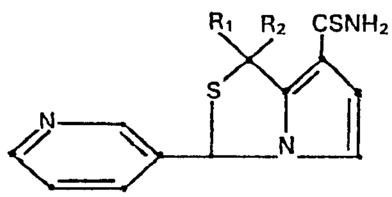

in which $R_1$ and $R_2$ are defined as in Claim 1, and the product obtained is then isolated and optionally converted to an addition salt with an acid.

11. Pharmaceutical composition, characterized in that it contains as active product at least one compound according to Claim 1, optionally in combination with one or more diluents or adjuvants which are compatible and pharmaceutically acceptable.

**Claim for the contracting state:** AT

1. Process for preparing a 1H,3H-pyrrolo[1,2-c]-thiazole derivative of general formula:

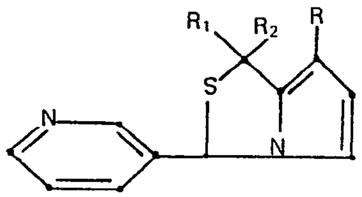

(I)

in which $R_1$ and $R_2$, wich may be identical or different, denote a hydrogen atom or an alkyl radical and
a) either R denotes a cyano or alkylcarbony radical,
b) or R denotes a radical of general formula:

$$-CON<^{R_3}_{R_4} \qquad (II)$$

in which
— either $R_3$ denotes a hydrogen atom and $R_4$ denotes an amino, alkylamino, dialkylamino, phenylamino or diphenylamino radical,
— or $R_3$ and $R_4$, which may be identical or different, denote a hydrogen atom or an alkyl radical containing 1 to 5 carbon atoms or an optionally substituted radical,
— or $R_3$ denotes a hydrogen atom and $R_4$ denotes a pyridyl radical or an alkyl radical containing 1 to 5 carbon atoms, substituted with a carboxy, amino, alkylamino, dialkylamino, morpholino, piperidino or 1-pyrrolidinyl radical, a 1-piperazinyl radical (optionally substituted at position 4 with an alkyl or pyridyl radical or an optionally substituted phenyl or optionally substituted benzyl radical), an optionally substituted phenyl radical or a pyridyl or imidazolyl radical,
or $R_3$ and $R_4$ together form an imidazolyl radical or a 5- or 6-membered heterocyclic ring which can contain in addition another hetero atom such as oxygen, sulphur or nitrogen, and is optionally substituted with an alkyl, alkyloxycarbonyl, hydroxyalkyl, aminoalkyl, alkylaminoalkyl, dialkylaminoalkyl, pyridyl, pyrimidinyl or pyrazinyl radical, or an optionally substituted phenyl or optionally substituted benzyl radical,
c) or R denotes a radical of general formula:

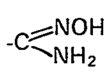 (III) or 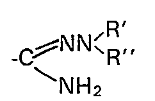 (IV)

in which R′ and R″, which may be idendical or different, denote an alkyl radical,
with the understanding that, in the above definitions, the alkyl radicals and alkyl portions are straight-chain or branched and contain, except where otherwise stated, 1 to 4 carbon atoms, that the substituted phenyl and benzyl radicals are phenyl and benzyl radicals bearing a halogen atom or an alkyl, alkyloxy, alkylthio, trifluoromethyl or dialkylamino radical, and that the invention also relates to the tautomeric forms of the abovementioned products when R denotes a radical of general formula (III) or (IV), as well as its addition salts with acids and, where appropriate, its metal salts and addition salts with nitrogenous bases, characterized in that
A - for the preparation of a product of general formula (I) in which R denotes a cyano radical and $R_1$ and $R_2$ are defined as above, 2-chloroacrylonitrile is reacted with a product of general formula:

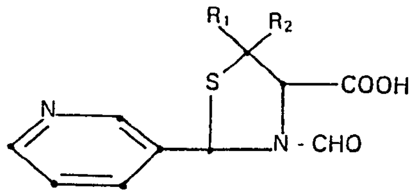

in which $R_1$ and $R_2$ are defined as above, and the product is then isolated and optionally converted to an addition salt with an acid, or in that
B - for the preparation of a product of general formula (I) in which $R_1$ and $R_2$ are defined as above and R denotes a radical of general formula:

$$-CON<^{R_3}_{R_4}$$

in which $R_3$ and $R_4$ together form an imidazolyl ring, N,N′-carbonyldiimidazole is reacted with an acid of general formula:

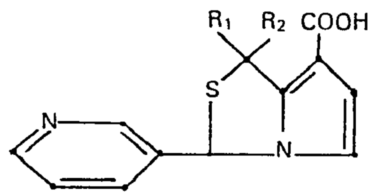

in which $R_1$ and $R_2$ are defined as above, and the product obtained is then isolated and optionally converted to an addition salt with an acid, or in that
C - for the preparation of a product of general formula (I) in which $R_1$ and $R_2$ are defined as above and R denotes a radical of general formula:

$$-CON<^{R_3}_{R_4}$$

in which $R_3$ and $R_4$ are defined as at b) above, with the exception of the possibility of $R_3$ and $R_4$ together forming an imidazolyl radical, ammonia or a product of general formula:

$$HN<^{R_3}_{R_4}$$

in which $R_3$ and $R_4$ are defined as at b) above, with the exception of the possibility of $R_3$ and $R_4$ together forming an imidazolyl ring, is reacted with an acid of general formula:

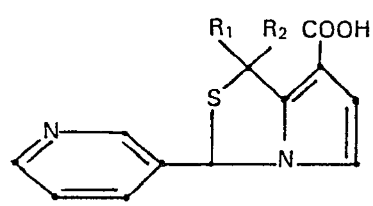

in which $R_1$ and $R_2$ are defined as above, the product obtained is then isolated and optionally converted to an addition salt with an acid or, where appropriate, to a metal salt or an addition salt with a nitrogenous base, or in that
D - for the preparation of a product of general formula (I) in which $R_1$ and $R_2$ are defined as above and R denotes a radical of general formula:

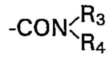

in which $R_3$ and $R_4$ each denote a hydrogen atom, a nitrile of general formula:

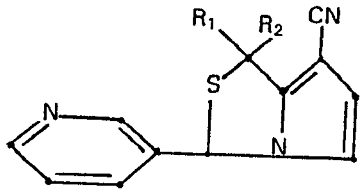

in which $R_1$ and $R_2$ are defined as above, is hydrolysed by any method known to those versed in the art, and the product obtained is then isolated and optionally converted to an addition salt with an acid, or in that

E - for the preparation of a product of general formula (I) in which $R_1$ and $R_2$ are defined as above and R denotes a radical of general formula:

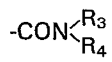

in which $R_3$ and $R_4$ each denote a hydrogen atom, 2-chloroacrylamide is reacted with an acid of general formula:

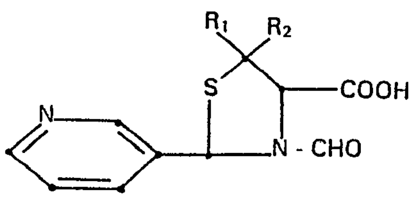

in which $R_1$ and $R_2$ are defined as above, and the product obtained is then isolated and optionally converted to an addition salt with an acid, or in that

F - for the preparation of a product of general formula (I) in which $R_1$ and $R_2$ are defined as above and R denotes an acetyl radical, the ethoxymagnesium derivative of ethyl malonate is reacted with a product of general formula:

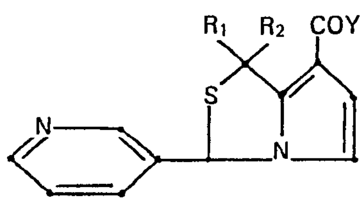

in which $R_1$ and $R_2$ are defined as above and Y denotes a halogen atom, and the product obtained is then hydrolysed and decarboxylated and optionally converted to an addition salt with an acid, or in that

G - for the preparation of a product of general formula (I) in which $R_1$ and $R_2$ are defined as above and R denotes an alkylcarbonyl radical, an organomagnesium derivative of general formula:

R''' Mg X

in which R''' denotes an alkyl radical and X denotes a halogen atom, is reacted with a nitrile of general formula:

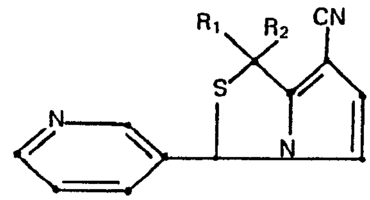

in which $R_1$ and $R_2$ are defined as above, and the product obtained is then hydrolysed and the final product optionally converted to an addition salt with an acid, or in that

H - for the preparation of a product of general formula (I) in which $R_1$ and $R_2$ are defined as above and R denotes a radical of general formula:

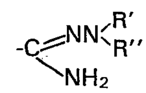

in which R' and R'' are defined as above, a hydrazine of general formula:

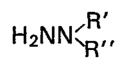

in which R' and R'' are defined as above, is reacted with a product of general formula:

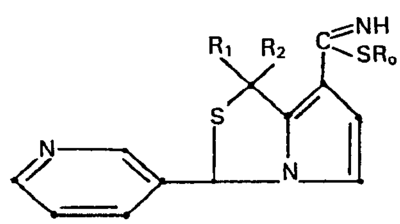

in which $R_1$ and $R_2$ are defined as above and $R_o$ denotes an alkyl radical, and the product obtained is then isolated and optionally converted to an addition salt with an acid, or in that

I - for the preparation of a product of general formula (I) in which $R_1$ and $R_2$ are defined as above and R denotes a radical of formula:

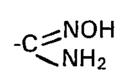

hydroxylamine is reacted with a product of general formula:

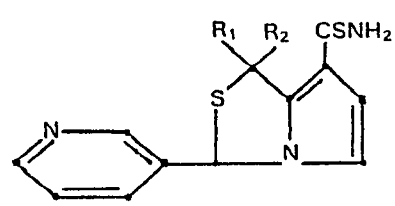

in which $R_1$ and $R_2$ are defined as above, and the product obtained is then isolated and optionally converted to an addition salt with an acid.